# EUROPEAN PATENT SPECIFICATION

(11) **EP 1 709 182 B1**
(45) Date of publication and mention of the grant of the patent: **27.10.2010**
(21) Application number: 05707058.3
(22) Date of filing: 28.01.2005
(51) Int. Cl.: C12N 15/81, C12N 1/15, C12N 1/19

(54) **GENERATION OF RECOMBINANT GENES IN SACCHAROMYCES CEREVISIAE**
ERZEUGUNG REKOMBINANTER GENE IN SACCHAROMYCES CEREVISIAE
GENERATION DE GENES RECOMBINANTS DANS SACCHAROMYCES CEREVISIAE

(30) Priority: 30.01.2004 EP 04360008
(43) Date of publication of application: 11.10.2006
(73) Proprietor: Mixis France S.A., 75014 Paris (FR)
(72) Inventor: SMITH, Kathleen, F-75015 Paris (FR); BORTS, Rhona, Leicester, LE79LN (GB)
(74) Representative: Crawley, Karen Anne
(86) International application number: PCT/EP2005/000841
(87) International publication number: WO 2005/075654

(56) References cited:
- PALUH J L ET AL: "MUTATIONAL ANALYSIS OF THE GENE FOR SCHIZOSACCAROMYCES POMBE RNASE MRP RNA, MRPL, USING PLASMID SHUFFLE BY COUNTERSELECTION ON CANAVANINE" YEAST, CHICHESTER, SUSSEX, GB, vol. 12, no. 14, November 1996 (1996-11), pages 1393-1405, XP001000809 ISSN: 0749-503X
- ABECASSIS V. ET AL.: "High efficiency family shuffling based on multi-step PCR and in vivo Dna recombination in yeast: statistical and functional analysis of a combinatorial library between human cytochrome P450 1A1 and 1A2." NUCLEIC ACIDS RESEARCH, vol. 28, no. 20, 15 October 2000 (2000-10-15), pages E88-E88, XP002331294

## Description

The present invention relates in general to methods for generating and detecting recombinant DNA sequences in *Saccharomyces cerevisiae* and plasmids and *S. cerevisiae* cells used for conducting the inventive methods.

DNA sequences for which these methods are relevant include protein-encoding and non-coding sequences; they may also consist of larger continuous stretches that contain more than a single coding sequence with intervening non-coding sequences, such as those that as may belong to a biosynthetic pathway.

The microbial and enzymatic production of substances such as enzymes and other proteins is an important economical topic. Enzymes are biocatalytically active proteins not only responsible for the metabolism of natural compounds and organisms, but also utilized for the industrial production of natural and non-natural compounds. Enzymes or those compounds produced by the help of enzymes can be used for the production of drugs, cosmetics, foodstuffs, etc. However, the industrial use of enzymes has been greatly hindered by their target specifity and the specific conditions under which they can function. Other proteins have therapeutic applications in the fields of human and animal health. Important classes of medically important proteins include cytokines and growth factors.

Proteins, enzymes, and pathways with novel or improved functions and properties can be obtained either by searching among largely unknown natural species or by improving upon currently known natural proteins or enzymes. The latter approach may be more suitable for creating properties for which natural evolutionary processes are unlikely to have been selected.

One promising strategy to create such novel desirable properties and to redesign enzymes, other proteins, non-coding sequences or pathways is by directed molecular evolution. Conventionally, as direct evolution of DNA sequences has been achieved with such techniques as site-directed mutagenesis, multi-site or cassette mutagenesis, random mutagenesis, and error prone PCR. Recently, gene shuffling approaches to optimize or fine-tune the properties of enzymes or proteins have attracted much attention. These directed evolutionary techniques can produce enzymes that can improve existing technology, produce novel products and expand the capabilities of synthetic chemistry.

A number of different mutagenesis methods exist, such as random mutagenesis, site-directed mutagenesis, oligonucleotide cassette mutagenesis, or point mutagenesis by error-prone PCR. Random mutagenesis, for example, entails the generation of a large number of randomly distributed, nucleotide substitution mutations in cloned DNA fragments by treatment with chemicals such as nitrous acid, hydrazine, etc. Error-prone PCR has been developed to introduce randorn point mutations into cloned genes. Modifications that decrease the fidelity of the PCR reaction include increasing the concentration of MgCl₂, adding MnCl₂, or altering the relative concentrations of the four dNTPs.

These traditional mutagenesis methods focus on the optimization of individual genes having discrete and selectable phenotypes. The general strategy is to clone a gene, identify a discrete function for the gene, establish an assay by which it can be monitored, mutate selected positions in the gene and select variants of the gene for improvement in the known function of the gene. A variant having improved function can then be expressed in a desired cell type. Repetitive cycles of mutagenesis methods can be carried out to obtain desirable enzyme properties.

Each of these conventional approaches has an implicit sequence search strategy. The strategies employed in the above techniques of sequence searching are very different. Performing a saturating site-directed mutagenesis search involves a process of installing every possible permutation at a site of interest. For a protein, this procedure consists of replacing an amino acid at a site of interest with all 19 other amino acids and searching the resultant library for improved mutants. In sequence space terms this means that a very small region has been searched very thoroughly. In comparison, cassette-mutagenesis inserts a random peptide sequence in a specific region of a protein, giving a less thorough sampling of a larger, defined region of sequence space. Error-prone PCR involves repeated copying of a sequence, with the introduction of a low but significant number of errors. In this case, a sparse sampling of a less defined region of sequence space is achieved. In each of these strategies, the best mutant obtained in each round of selection is used to initiate the next round.

However, traditional mutagenesis approaches for evolving new properties in enzymes have a number of limitations. First, they are only applicable to genes or sequences that have been cloned and funcfunctionally characterized. Second, these approaches are usually applicable only to genes that have a discrete function. Therefore, multiple genes that cooperatively confer a single phenotype usually cannot be optimized in this manner. Finally, these approaches can only explore a very limited number of the total number of permutations, even for a single gene. In view of these limitations, conventional mutagenesis approaches are inadequate for improving cellular genomes with respect to many useful properties. For example, improvements in the capacity of a cell to express a protein might require alterations in transcriptional efficiency, translation and post-translational modifications, secretion or proteolytic degradation of a gene product. It therefore might be necessary to modify additional genes having a role in one or more of these cellular mechanisms in order to express a protein with new properties. Attempting to individually optimize all of the genes having such function would be a virtually impossible task.

Most of the problems associated with conventional mutagenesis approaches can be overcome by gene shuffling approaches. Gene shuffling entails randomly recombining different sequences of functional genes, enabling the molecular mixing of naturally similar or randomly mutated genes. DNA or gene shuffling, or variations of these techniques, have been used to improve the activity, stability, folding, and substrate recognition properties of enzymes. In comparison to conventional mutagenesis approaches with gene shuffling, the probability of obtaining mutants with improved phenotype is significantly higher. Gene shuffling is fundamentally different from conventional strategies in that it recombines favorable mutations in a combinatorial fashion. It therefore will search much larger regions of sequence space much more sparsely and with a bias towards producing functional sequences. It also allows more beneficial mutations from each round of selection to be retained in the next round because it allows sequence information to be contributed from more than one source. Whereas conventional strategies also allow for the fixation of negative mutations, this is not the case for gene shuffling approaches. Therefore, it is not surprising that gene shuffling strategies have yielded much more dramatic results.

DNA shuffling techniques are described by Paluh J L et al: "Mutational Analysis of the Gene for Schizosac Caromyces Pombe RNASE MRP, RNA, MRPL, Using Plasmid Shuffle by Counterselection on Canavanine" Yeast, Chichester, Sussex, GB, vol. 12, no. 14, November 1996 (1996-11), pages 1393-1405 and also by Abecassis V. et al: "High efficiency family shuffling based on multi-step PCR and in vivo DNA recombination in yeast: statistical and functional analysis of a combinatorial library between human cytochrome P450 1A1 and 1A2." Nucleic Acids Research, vol. 28, no.20, 15 October 2000 (2000-10-15), pages E88-E88.

DNA or gene shuffling approaches are based on recombination events between regions with a certain homology or between stretches of identity. A key organism used in experiments to examine genetic recombination in eukaryotes has been the budding yeast Saccharomyces cerevisiae. The study of these processes in a simple, unicellular organism has the obvious advantage of the ease of manipulation of DNA sequences and the possibility of studying specific recombination events induced synchronously in a large proportion of cells. Furthermore, over the last few decades a wealth of expertise has been accumulated both in the fermentation technology and the basic genetics of this organism, which is at the best studied eukaryote at the molecular level. Due to its non-pathogenic character, its secretion proficiency and its glycosylation potential, S. cerevisiae is a preferred host organism for gene cloning and gene expression. Therefore, the technical problem underlying the present invention is to provide methods and means for the generation of recombinant mosaic genes in Saccharornyces cerevisiae.

The present invention solves this underlying technical problem by providing a process for generating and detecting recombinant DNA sequences in Saccharomyces cerevisiae comprising the steps of:
a) generating first diploid S. cerevisiae cells bearing in a defined locus of their genome a first recombination cassette comprising a first DNA sequence to be recombined, which is flanked by at least a first and a second marker sequence, in an allelic position a second recombination cassette comprising a second DNA sequence to be recombined, which is flanked by at least a third and a fourth marker sequence,
b) inducing the sporulation of the first diploid cells obtained in a)
   and
c) isolating haploid cells containing recombination cassettes in which first recombined DNA sequences are flanked by the first and fourth marker sequences, and haploid cells containing recombination cassettes in which second recombined DNA sequences are flanked by the second and the third marker sequences.

The present invention provides a yeast-based system to screen for recombination events between at least two diverging DNA sequences. The system is based on the sexual reproductive cycle of S. cerevisiae, which alternates between a haploid phase and a diploid phase. In the first step of the inventive process, diploid S. cereivisiae cells are generated, which are heterozygous for these recombination substrates. The DNA sequences to be recombined are integrated in the genome of the diploid S. cerevisiae cells at allelic positions. Each DNA sequence to be recombined is integrated in the form of a recombination cassette, which comprises besides this DNA sequence at least two marker sequences that flank the DNA sequence, whereby the two recombination cassettes comprise at least four different marker sequences.

The heterozygous diploid cells thus obtained are then grown under conditions which induce the processes of meiosis and spore formation. Meiosis is generally characterized by elevated frequencies of genetic recombination, which is initiated via the formation and subsequent repair of double-strand breaks (DSBs) induced early in meiosis I prophase. Yeast meiotic cells are therefore of particular interest because they experience high levels of recombination as a result of the genome-wide induction of DSBs. Thus the products of a first round of meiosis, which are haploid cells or spores for each meiosis event four produced by a parental diploid cell, can contain recombined DNA sequences due to recombination between the two diverged DNA sequences.

Recombination between the two diverging DNA sequences during meiosis can also lead to an exchange of the flanking marker sequences. Therefore, the present process allows a rapid and simple identification of recombined DNA sequences by the selection of individual cells or molecules in which an exchange of marker sequences flanking a recombination substrate has taken place. The recombinants obtained after the first round of meiosis are therefore characterized in that they contain and/or express at least one marker sequence of the first recombination cassette and at least one marker sequence of the second recombination cassette. In particular, recombinant spores can contain the first marker sequence of the first recombination cassette and the fourth marker sequence of the second recombination cassette or the second marker sequence of the first recombination cassette and the third marker sequence of the second recombination cassette, whereby both types of recombinant spores contain besides this different marker combination also different recombinant DNA sequences. Both types of spores containing recombinant sequences can easily be selected and distinguished under conditions that permit selection for the new recombinant marker configurations produced by recombination during meiosis.

The inventive process can be conducted either in wild-type or mismatch repair-defective S. cerevisiae cells. The processes by which damaged DNA is repaired and the mechanisms of genetic recombination are intimately related, and it is known that the mismatch repair machinery has inhibitory effects on the recombination frequency between divergent sequences, i.e. homeologous recombination. Mutations of the mismatch repair system therefore greatly enhance the overall frequency of recombination events in yeast. On the other hand, it is known that wild-type S. cerevisiae cells have a mismatch repair-dependent recombination mechanism, which is based on distantly spaced mismatches in two recombination substrates. Depending on the DNA sequences to be recombined, either wild-type or mismatch repair-defective S. cerevisiae cells can be used to obtain recombined sequences.

The inventive process has the advantage that it is iterative, i.e. it allows further rounds of recombination. The products of the first round of meiosis, i.e. haploid cells of opposite mating types which comprise different recombined DNA sequences, are mated again to obtain diploid cells which are heterozygous for recombined DNA sequences. In the diploid cells thus obtained meiosis is again induced, whereby the recombined DNA sequences are once again recombined, leading again to an exchange of the two markers flanking each recombination substrate. The new haploid recombinants obtained after the second meiosis can now be easily identified by the joint expression of either those marker genes which flanked the first DNA sequence in the original first recombination cassette or those marker genes which flanked the second DNA sequence in the original second recombination cassette.

In a preferred embodiment of the invention therefore haploid cells containing a recombination cassette with the first recombined DNA sequences obtained in the first round of the inventive process are mated with haploid cells containing recombination cassettes with the second recombined DNA sequences obtained in the first round of the inventive process in order to generate second diploid cells. In the thus obtained second diploid cell sporulation is induced, resulting in the generation of haploid cells. In the next steps haploid cells containing recombination cassettes in which third recombined DNA sequences are flanked by at least the first and second marker sequences and haploid cells containing recombination cassettes in which fourth recombined DNA sequences are flanked by at least the third and fourth marker sequences are isolated.

Further recombined DNA sequences can be generated by subjecting the haploid cells containing third and fourth recombined DNA sequences to one or more further cycles of mating and meiosis/sporulation. After each round of recombination, recombinants are either identified by the joint presence of at least one marker sequence that flanked the first recombination substrate and at least one marker sequence that flanked the second recombination substrate or by the joint presence of the two markers that flanked the first or the second DNA sequence in the starting recombination substrates.

Therefore, an advantageous feature of the present process is that it is iterative: recombinant haploid progeny is selected individually or en masse and mated to one another, the resulting diploids are sporulated anew, and their progeny spores are subjected to appropriate selection conditions to identify new recombination events. With the inventive process a large library of recombined, mutated sequences can be easily generated, and variants that have acquired a desired function can then be identified by using an appropriate selection or screening system.

In a preferred embodiment of the invention the first diploid S. cerevisiae cell is generated by simultaneously or sequentially transforming a diploid S. cerevisiae cell with a DNA molecule comprising the first recombination cassette and a DNA molecule comprising the second recombination cassette and optionally allowing the integration of the two recombination cassettes into allelic positions on natural chromosomes of the S. cerevisiae genome. The DNA molecules used can also be for example yeast artificial chromosomes (YAC). YACs are **characterized in that** they are linear DNA molecules that contain all the sequences necessary for stable maintenance in the yeast cell, such as a centromere, DNA replication origin and telomeres as well as yeast selectable markers. Upon introduction into a yeast cell YACs behave similar to natural chromosomes and therefore can be considered as part of the yeast genome. In the context of the present invention the term "genome" includes the whole of all hereditary components present within a cell, which are stably maintained and inherited. In case YACs are used as DNA molecules for introduction the first and second recombination cassettes into diploid S. cerevisiae cells it is not necessary to integrate the two recombination cassettes into allelic positions in natural chromosomes. In the case in which recombination cassettes are introduced into natural chromosomes, it is possible to use a cloning vehicle, for example a plasmid, from which a fragment bearing the recombination cassettes can be liberated. Preferably the two respective marker sequences of the two recombination cassettes are flanked by targeting sequences which are homologous to a defined locus of the S. cerevisiae genome. Alternatively, a DNA molecule can be used which does not contain a replication origin. In this case the DNA molecules must be able to integrate into a component of the genome and therefore contain targeting sequences which are homologous to a defined locus of the S. cerevisiae genome.

In another preferred embodiment of the invention the first diploid S. cerevisiae cells are generated by fusing haploid cells bearing in a locus of their genome the first recombination cassette with S. cerevisiae haploid cells bearing in an allelic position of their genome the second recombination cassette.

In still another preferred embodiment of the invention the first diploid S. cerevisiae cells are generated by mating haploid cells bearing in a locus of their genome the first recombination cassette with S. cerevisiae haploid cells of opposite mating type bearing in an allelic position of their genome the second recombination cassette.

In the context of the present invention the terms "mating" and "fusing" denote either the purposeful or the random combination of two haploid cells containing different recombination cassettes. A purposeful mating or fusing of two haploid cells occurs, when two selected and//or isolated haploid cells of opposite mating type with desired properties are brought into contact under conditions stimulating mating and fusing, respectively. The two haploid cells can be derived from the same library of cells, which for example contain DNA sequences to be recombined or already recombined DNA sequences, or from different libraries of cells, which for example contain DNA sequences to be recombined or already recombined DNA sequences.

A random mating or fusing of two haploid cells can occur, when a plurality of different haploid cells are brought into contact under conditions stimulating mating and fusing, respectively. The plurality of haploid cells can be derived from the same library of cells, which for example contain DNA sequences to be recombined or already recombined DNA sequences, or from different libraries of cells, which for example contain DNA sequences to be recombined or already recombined DNA sequences.

The inventive process for generating and detecting recombined DNA sequences has the advantage that more than two diverging sequences can be recombined. If, for example, four diverging DNA sequences shall be recombined, then in the first step of the present process two different sets of diploid S. cerevisiae cells can be generated. For example, a first set of diploid cells can be generated by mating or fusing haploid cells comprising a first and a second DNA sequence to be recombined and a second set of diploid cells can be generated by mating or fusing haploid cells comprising a third and a fourth DNA sequence to be recombined. After sporulation of the two sets of diploid cells haploid cells obtained from the first diploid cell set that contain recombined DNA sequences due to recombination between the first and the second DNA sequence, are mated with appropriate haploid cells obtained from the second diploid cell set that contain recombined DNA sequences due to recombination between the third and the fourth DNA sequences. The products of this mating are diploid cells which after sporulation give rise to haploid cells bearing recombined DNA sequences which comprise regions of the first DNA sequence, the second DNA sequence, the third DNA sequence and the fourth DNA sequence. If, for example, three diverging DNA sequences shall be recombined, in the first step of the present process diploid S. cerevisiae cells are generated by, for example, mating or fusing haploid cells comprising a first and a second DNA sequence to be recombined. After sporulation of these diploid cells, the haploid cells thus obtained, which contain recombined DNA sequences due to recombination between the first and the second DNA sequences, can be fused or mated with haploid cells comprising a third DNA sequence to be recombined. The products of this mating are diploid cells which after sporulation give rise to haploid cells bearing recombined DNA sequences which comprise regions of the first DNA sequence, the second DNA sequence and the third DNA sequence. In this way, five, six or more diverging DNA sequences can also be recombined.

In a preferred embodiment haploid S. cerevisiae cells bearing the first or second recombination cassette are generated by:
a) inserting the first DNA sequence to be recombined between the first and the second marker sequence located adjacently on a first cloning vehicle and inserting the second DNA sequence to be recombined between the third and the fourth marker sequence located adjacently on a second cloning vehicle, whereby the respective two marker sequences are flanked by targeting sequences which are homologous to a defined locus of the S. cerevisiae genome,
b) excising from the cloning vehicles obtained in a) the first recombination cassette and the second recombination cassette with flanking targeting sequences, respectively, whereby each excised fragment comprises the DNA sequence to be recombined, which is flanked by the respective two marker sequences and by targeting sequences,
c) transforming the excised fragments obtained in b) separately into S. cerevisiae diploid cells, whereby the targeting sequences direct the integration of the cassettes into that locus to which they are homologous, in order to obtain diploid cells heterozygous for the first cassette, or the second cassette,
d) inducing separately the sporulation of the heterozygous diploid cells obtained in c) and
e) isolating haploid cells containing the first cassette flanked by the first and second marker sequences and separately haploid cells containing the second cassette flanked by the third and the fourth marker sequences.

In a preferred embodiment of the invention the respective two marker sequences in the first or second cloning vehicle are flanked by targeting sequences which are homologous to the *BUD31-HCM1* locus on chromosome III of the S. cerevisiae genome and which direct the integration of the excised cassettes into that locus.

In a preferred embodiment of the invention the cloning vehicle used for cloning the recombination cassettes is a plasmid. "Plasmid" means an extrachromosomal element which can autonomously replicate. The plasmid is physically unlinked to the genome of the cell wherein it is contained. Most plasmids are double-stranded circular DNA molecules. In another embodiment the cloning vehicle is an YAC.

In particular it is preferred to use as the first cloning vehicle, in which the first recombination cassette is cloned, plasmid pMXY9. Plasmid pMXY9 comprises the *URA3* marker gene and the *CAN1* marker gene. In this plasmid the two marker genes are adjacently located. Between the two marker genes are arranged several restriction sites, in particular recognition sites for the restriction enzymes *Sma*I, *Xba*I, *Bgl*II and *Pac*I, for inserting a DNA sequence to be recombined. The two marker sequences are flanked by targeting sequences homologous to the *BUD31-HCM1* locus on chromosome III of the S. cerevisiae genome.

Furthermore, it is preferred to use as the second cloning vehicle, in which the second recombination cassette is cloned, plasmid pMXY12. Plasmid pMXY12 comprises the *TRP1* marker gene and the *CYH2* marker gene. In this plasmid the two marker genes are adjacently located. Between the two genes are arranged several restriction sites, in particular recognition sites for the restriction enzymes *Spe*I, *Sma*I and *Pac*I, for inserting a DNA sequence to be recombined. The two marker sequences are flanked by targeting sequences homologous to the *BUD31-HCM1* locus on chromosome III of the S. cerevisiae genome.

In a preferred embodiment of the invention the diploid cells used for transformation of the excised recombination cassette are auxotrophic for at least two nutritional factors and resistant to at least two antibiotics. Preferably, the diploid cells are homozygous for the *ura3-1* allele and the *trp1-1* allele, which renders the cells auxotrophic for uracil and tryptophan, respectively. Furthermore it is preferred that the diploid cells used for transformation are homozygous for the *can1-100* allele and the *cyh2R* allele, which renders them resistant to canavanine and cycloheximide, respectively.

In particular it is preferred, that diploid cells of the S. cerevisiae strain MXY47 are used for transformation, which are homozygous for the alleles *ura3-1, trp1-1, can1-100* and *cyh2R* and heterozygous for the *msh2::KanMX* mutation. When diploid cells of the strain MXY47 are used for the transformation with the excised first or second fragments bearing recombination cassettes and their flanking targeting sequences, then transformants obtained can be sporulated to yield haploid wild type or *msh2* segregants that bear the respective recombination cassette.

According to the invention it may be preferred to use S. cerevisiae cells which have a functional mismatch repair system for the inventive process. The mismatch repair system belongs to the largest contributors to avoidance of mutations due to DNA polymerise errors in replication. Mismatch repair also promotes genetic stability by editing the fidelity of genetic recombination. It is known that, therefore, the mismatch repair machinery has a somewhat inhibitory effect on recombination between diverged sequence. However, in a normal S. cerevisiae diploid another aspect of mismatch repair, termed mismatch repair-dependent recombination, was detected (Borts and Haber, Science, 237 (1987), 1459-1465). It is thought that the mismatch repair of widely spaced mismatches such as in diverged sequences leads to new double-strand breaks that can in turn stimulate a second round of (mismatch repair-dependent) recombination. In certain circumstances, in particular, when it is known that the two recombination substrates used have widely spaced base differences, it is therefore useful to employ S. cerevisiae cells with a functional mismatch repair system for conducting the inventive process.

In another preferred embodiment of the invention, S. cerevisiae cells that are deficient in the mismatch repair system are used. In S. cerevisiae several genes have been identified whose products share homology with bacterial mismatch repair proteins, including six homologues of the MutS protein, i.e. Msh1, Msh2p, Msh3p, Msh4, Msh5 and Msh6p, and four homologues of the MutL protein, i.e. Mlh1p, Mlh2p, Mlh3p, and Pms1. It is known that in particular the *PMS1* and *MSH2* genes set up a barrier to the recombination of diverged sequences. Therefore, in *msh2* and *pms1* mutants, meiotic recombination between diverged sequences is increased, relative to the frequency of recombination in wild type cells.

In the context of the present invention the term "deficient in the mismatch repair system" means that the mismatch repair system (MMR) of a cell is transiently or permanently impaired. MMR deficiency of a cell or an organism can be achieved by any strategy that transiently or permanently impairs the mismatch repair including but not limited to a mutation of one or more genes involved in mismatch repair, treatment with an agent like UV light, which results in a global impairment of MMR, treatment with an agent like 2-aminopurin or a heteroduplex containing an excessive amount of mismatches to transiently saturate and inactivate the MMR system and inducible expression or repression of one or more genes involved in the mismatch repair, for example via regulatable promoters, which would allow for transient inactivation, i.e. during meiosis, but not during vegetative growth.

In a preferred embodiment of the invention the mismatch repair deficiency of the S. cerevisiae cell is due to a mutation of at least one gene involved in the MMR. In a preferred embodiment the S. cerevisiae cells are deficient in the *MSH2* gene. Preferably, diploid cells are homozygous for the *msh2* allele, in which the *MSH2* coding sequences are replaced by the KanMX construct.

In the context of the present invention the term "recombination cassette" refers to a DNA sequence comprising at least one recombination substrate or one DNA sequence to be recombined, which is flanked by at least two different marker sequences. The first and the second recombination cassette differ in the DNA sequences to be recombined and in the flanking marker sequences, such that any pair of recombination cassettes comprises two different DNA sequences to be recombined and at least four different flanking marker sequences.

In a preferred embodiment of the invention both the first and the second recombination cassettes are generated by inserting the respective DNA sequences to be recombined between two marker sequences that are closely located on a cloning vehicle and which in turn are surrounded by targeting sequences that are homologous to a defined locus of the S. cerevisiae genome. The targeting sequences therefore can direct the integration of an excised fragment containing a recombination cassette into this defined locus. The insertion of the DNA to be recombined between the two marker sequences is preferably effected by genetic engineering methods. In a preferred embodiment of the invention the two marker sequences in the cloning vehicle are flanked by targeting sequences which are homologous to the *BUD31-HCM1* locus on chromosome III of the S. cerevisiae genome. Therefore, the targeting sequences direct the integration of the excised fragments containing a recombination cassettes into that locus.

In the context of the present invention the terms "DNA sequences to be recombined" and "recombination substrate" mean any two DNA sequences that can be recombined as a result of meiotic recombination processes, whereby recombination between these sequences can be due to homologous or non-homologous recombination.

Homologous recombination events of several types are characterized by the base pairing of a damaged DNA strand with a homologous partner, where the extent of interaction can involve hundreds of nearly perfectly matched base pairs. The term "homology" denotes the degree of identity existing between the sequence of two nucleic acid molecules. In contrast, illegitimate or non-homologous recombination is characterized by the joining of ends of DNA that share no or only a few complementary base pairs. In yeast, non-homologous repair and recombination events occur at significantly lower frequencies than homologous recombination events.

The first and second DNA sequences to be recombined are diverging sequences, i.e. sequences, which are not identical but show a certain degree of homology. This means, that the DNA sequences to be recombined diverge by at least one nucleotide. Preferably the DNA sequences to be recombined are sequences that share at least one or more homologous regions, which can be very short. The homologous regions should comprise at least 5-10 nucleotides, preferably more than 20-30 nucleotides, more preferred more than 30-40 nucleotides and most preferred more than 50 nucleotides. In a preferred embodiment of the invention the first and the second DNA sequences to be recombined diverge by at least one nucleotide, in particular more than 0,1 %, preferably more than 5 % to more than 50 %. This means, that the first and second DNA sequences to be recombined can also diverge by 55%, 60%, 65 % or even more.

Recombination substrates or DNA sequences to be recombined can have a natural or synthetic origin. DNA sequences to be recombined therefore can be derived from any natural source including viruses, bacteria, fungi including S. cerevisiae, animals, plants and humans. In a preferred embodiment of the invention the first and the second DNA sequences to be recombined are derived from organisms other than S. cerevisiae.

In a preferred embodiment of the invention DNA sequences to be recombined are protein-encoding sequences, for example sequences encoding enzymes, which can be utilized for the industrial production of natural and non-natural compounds. Enzymes or those compounds produced by the help of enzymes can be used for the production of drugs, cosmetics, foodstuffs, etc. Protein-encoding sequences can also be sequences, which encode proteins, that have therapeutic applications in the fields of human and animal health. Important classes of medically important proteins include cytokines and growth factors. The recombination of protein coding sequences allows for the generation of new mutated sequences which code for proteins with altered, preferably improved functions and/or newly acquired functions. In this way it is possible, for example, to achieve improvements in the thermostability of a protein, to change the substrate specificity of a protein, to improve its activity, to evolve new catalytic sites and/or to fuse domains from two different enzymes. Protein coding DNA sequences to be recombined can include sequences from different species which code for the same or similar proteins that have in their natural context similar or identical functions. Protein coding DNA sequences to be recombined can include sequences from the same protein or enzyme family. Protein coding sequences to be recombined can also be sequences which code for proteins with different functions - for example, sequences that code for enzymes which catalyse different steps of a given metabolic pathway. In a preferred embodiment of the invention the first and the second DNA sequences to be recombined are selected from the group of gene sequences of the Oxa superfamily of B-lactamases.

In another preferred embodiment of the invention DNA sequences to be recombined are non-coding sequences such as sequences, which, for example, are involved within their natural cellular context in the regulation of the expression of a protein-coding sequence. Examples for non-coding sequences include but are not limited to promoter sequences, sequences containing ribosome binding sites, intron sequences, polyadenylation sequences etc. By recombining such non-coding sequences it is possible to evolve mutated sequences, which in a cellular environment result in an altered regulation of a cellular process - for example, an altered expression of a gene.

According to the invention a recombination substrate or DNA sequence to be recombined can of course comprise more than one protein coding sequence and/or more than one non-coding sequence. For example a recombination substrate can comprise one protein coding sequence plus one non-coding sequence or a combination of different protein coding sequences and different non-coding sequences. In another embodiment of the invention DNA sequences to be recombined therefore can consist of one or more stretches of coding sequences with intervening and/or flanking non-coding sequences. That means, the DNA sequence to be recombined can be for example a gene sequence with regulatory sequences at its 5'-terminus and/or an untranslated 3'-region or an mammalian gene sequence with an exon/intron structure. In still another embodiment of the invention DNA sequences to be recombined can consist of larger continuous stretches that contain more than a single coding sequence with intervening non-coding sequences, such as those that as may belong to a biosynthetic pathway or an operon. DNA sequences to be recombined can be sequences, which have already experienced one or more recombination events, for example homologous and/or non-homologous recombination events.

The recombination substrates can comprise non-mutated wild-type DNA sequences and/or mutated DNA sequences. In a preferred embodiment therefore it is possible to recombine wild-type sequences with already existing mutated sequences in order to evolve new mutated sequences.

In the context of the present invention the term "marker sequences" refers to unique DNA sequences that are positioned upstream or downstream of a recombination substrate or an already recombined DNA sequence in Saccharomyces cerevisiae cells. The presence of a marker sequence on the same molecule of DNA as the recombination substrate or already recombined DNA sequence, preferably in combination with another marker sequence positioned on the other side of the recombination substrate, allows that recombination substrate or already recombined DNA sequence to be recognized and selected for, whether by molecular or genetic methods. Therefore, in one preferred embodiment of the invention there must be one or more marker sequences upstream of each recombination substrate and one or more marker sequences downstream of each recombination substrate, such that in a cell heterozygous for two different recombination substrates, there are at least four different marker sequences altogether. This arrangement allows for the selection of crossovers involving recombination substrates. It also allows further rounds of recombination to be carried out in a iterative fashion. In another preferred embodiment of the invention more than one marker can be situated on each side of the recombination substrate. For example, additional markers can be introduced to increase the stringency of selection.

Marker sequences may comprise protein-encoding or non-coding DNA sequences. In a preferred embodiment of the invention the protein-encoding marker sequences are selected from the group consisting of nutritional markers, pigment markers, antibiotic resistance markers, antibiotic sensitivity markers and sequences that encode different subunits of an enzyme, which functions only, if both or more subunits are expressed in the same cell. In a further preferred embodiment of the invention the molecular non-coding marker sequences include but are not limited to primer recognition sites, i.e. sequences to which PCR primers anneal and which allow an amplification of recombinants, intron/exon boundaries, promoter sequences, downstream regulated gene sequences or restriction enzyme sites.

A "nutritional marker" is a marker sequence that encodes a gene product that can compensate an auxotrophy of an organism or cell and thus can confer prototrophy on that auxotrophic organism or cell. In the context of the present invention the term "auxotrophy" means that an organism or cell must be grown in a medium containing an essential nutrient which cannot be synthesized by the auxotrophic organism itself. The gene product of the nutritional marker gene promotes the synthesis of this essential nutrient missing in the auxotrophic cell. Therefore, upon expression of the nutritional marker gene it is not necessary to add this essential nutrient to the medium in which the organism or cell is grown, since the organism or cell has acquired prototrophy.

A "pigment marker" is a marker gene wherein the gene product is involved in the synthesis of a pigment which upon expression will stain that cell, in which the pigment marker is expressed. A cell without the pigment marker does not synthesize the pigment and is therefore not stained. The pigment marker therefore allows a rapid phenotypical detection of that cell containing the pigment marker.

An "antibiotic resistance marker" is a marker gene wherein the gene product confers upon expression to a cell, in which the expression of the antibiotic marker gene takes place, the ability to grow in the presence of a given antibiotic at a given concentration, whereas a cell without the antibiotic resistance marker cannot.

An "antibiotic sensitivity marker" is a marker gene wherein the gene product destroys upon expression the ability of a cell to grow in the presence of a given antibiotic at a given concentration.

In a preferred embodiment of the invention each of the gene products of the first and third marker sequences can compensate an auxotrophy of a S. cerevisiae cell. Preferably, the first marker sequence is *URA3,* the gene product of which can confer uracil prototrophy to a uracil auxotrophic S. cerevisiae cell. Preferably, the third marker sequence is *TRP1,* the gene product of which can confer tryptophan prototrophy to an tryptophan auxotrophic S. cerevisiae cell.

In another preferred embodiment of the invention the gene products of the second and fourth marker sequences confer sensitivity to an antibiotic to a S. cerevisiae cell which is resistant to that antibiotic. Preferably, the second marker sequence is *CAN1,* the gene product of which can confer to a canavanine resistant S. cerevisiae cell sensitivity to canavanine. Preferably, the fourth marker sequence is *CYH2,* the gene product of which can confer to a cycloheximide resistant S. cerevisiae cell sensitivity to cycloheximide.

In another preferred embodiment of the invention the marker sequences comprise annealing sites for PCR primers. Preferably, the first, second, third and fourth marker sequences are recognized by the primers KNS11, KNS28, KNS16, and KNS29.

In a preferred embodiment of the inventive process haploid cells containing recombination cassettes with either first, second, third or fourth recombined DNA sequences can be identified by PCR processes in order to detect the presence of the respective marker combination.

In another preferred embodiment of the inventive process haploid cells containing recombination cassettes with either first, second, third or fourth recombined DNA sequences are identified by plating the haploid cells on media that select for the presence on the same DNA molecule of the respective marker combination. This means that haploid cells containing first recombined DNA sequences are plated on a medium that selects for the presence of the first and the fourth marker sequences. Haploid cells containing second recombined DNA sequences are plated on a medium that selects for the presence of the second and the third marker sequences. Haploid cells containing third recombined DNA sequences are plated on a medium that selects for the presence of the first and the second marker sequences. Haploid cells containing fourth recombined DNA sequences are plated on a medium that selects for the presence of the third and the fourth marker sequences.

Another aspect of the present invention relates to a process of generating novel proteins, enzymes, pathways and non-coding sequences with novel or improved functions and properties, whereby known protein-coding sequences or known non-coding sequences are subjected one or more recombination rounds by using the inventive process for generating and detecting recombinant DNA sequences in S. cerevisiae.

Another aspect of the present invention relates to plasmid pMXY9. Plasmid pMXY9 comprises the *URA3* marker gene and the *CAN1* marker gene, which are located adjacently. Between the two marker gene a polylinker sequence, comprising several restriction sites for inserting a DNA sequence to be recombined, is arranged. The two markers are flanked by targeting sequences homologous to the *BUD31-HCM1* locus on chromosome III of the S. cerevisiae genome. The polylinker sequence between the two marker genes comprises restriction sites for the restriction enzymes *Sma*I, Xbal, *Bgl*II and *Pac*I.

Another aspect of the present invention relates to plasmid pMXY12. Plasmid pMXY12 comprises the *TRP1* marker gene and the *CYH2* marker gene. Between the two marker genes a polylinker sequence comprising several restriction sites for inserting a DNA sequence to be recombined is arranged. The two markers are flanked by targeting sequences homologous to the BUD31-HCM1 locus on chromosome III of the S. cerevisiae genome. The polylinker sequence comprises restriction sites for the restriction enzymes SpeI, SmaI and PacI.

The present invention relates also to the S. cerevisiae strain MXY47, **characterized in that** diploid cells thereof are homozygous for the alleles ura3-1, trp1-1, can1-100 and cyh2R and heterozygous for the msh2::KanMX mutation.

The present invention also relates to the E. coli strain JM101, containing plasmid pMXY9, and to E. coli strainDH5a, containing plasmid pMXY12.

Plasmids pMXY9 and pMXY12 and the Saccharomyces cerevisiae strain MXY47 were deposited on the 3^{rd} of January 2005 at the DSMZ (Deutsche Sammlung für Mikroorganismen und Zellkulturen GmbH, Mascheroderweg 1b, 38124 Braunschweig, Germany) under accession numbers DSM 17010, DSM 17011, and DSM 17026, respectively.

Another aspect of the present invention relates to a kit which can be used for conducting the inventive process for generating and detecting recombined DNA sequences in Saccharomyces cerevisiae. In a first embodiment the kit comprises at least a first container which contains cells of S. cerevisiae strain MXY47, a second container which contains cells of E. coli strain JM101 bearing plasmid pMXY9 and a third container containing cells of E. coli strain DH5a bearing plasmid pMXY12.

In a second embodiment the kit comprises at least a first container containing cells of S. cerevisiae strain MXY47, a second container containing DNA of plasmid pMXY9 and a third container containing DNA of plasmid pMXY12.

The present invention is illustrated by the following sequence listing, figures and example.

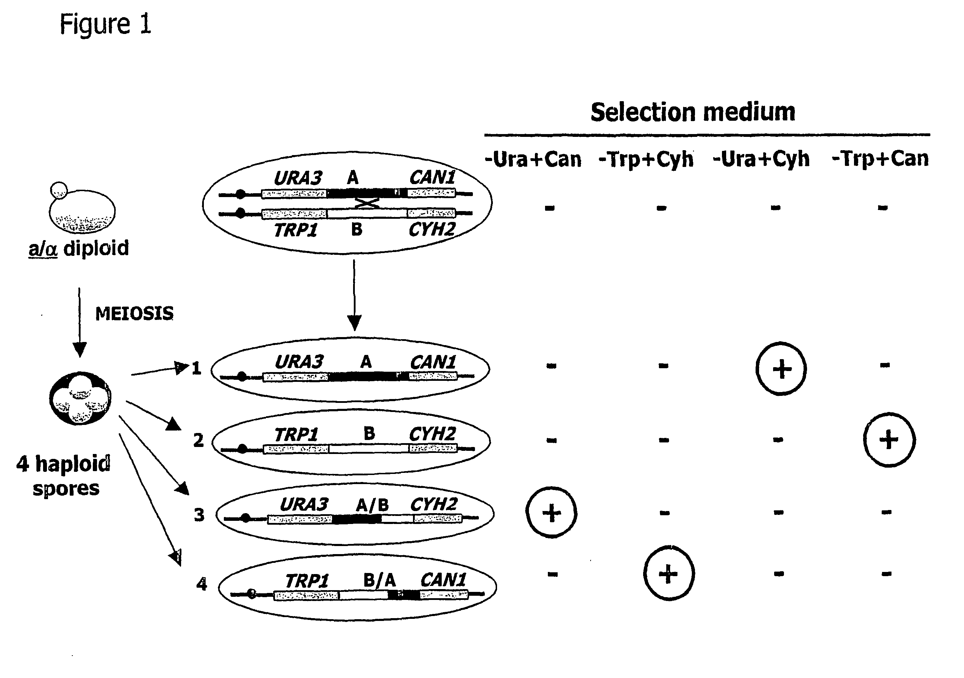

Figure 1 shows a schematic of the selection system for the selection of recombinants on defined media. Diploid parental cells heterozygous for recombination cassettes - here, recombination substrate A, flanked by the *URA3* and *CAN1* genes, and recombination substrate B, flanked by the *TRP1* and *CYH2* genes - are induced to undergo meiosis. Spores are plated on medium lacking uracil and containing canavanine (-Ura+Can) and on medium lacking tryptophan and containing cycloheximide (-Trp+Cyh) to select for recombinant cells 3 and 4, in which a crossover involving the recombination substrates A and B has taken place, as indicated by (+). Parental diploids and non-recombinant haploids 1 and 2 cannot grow on either of these media, as indicated by (-). A subsequent round of meiosis may use recombinants 3 and 4 to construct a new diploid, which when sporulated yields new recombinant cells bearing the same flanking marker configurations as those shown in cells 1 and 2. Recombinant spore colonies with these configurations can be selected on medium lacking uracil and containing cycloheximide (-Ura+Cyh), and on medium lacking tryptophan and containing canavanine(-Trp+Can), respectively.

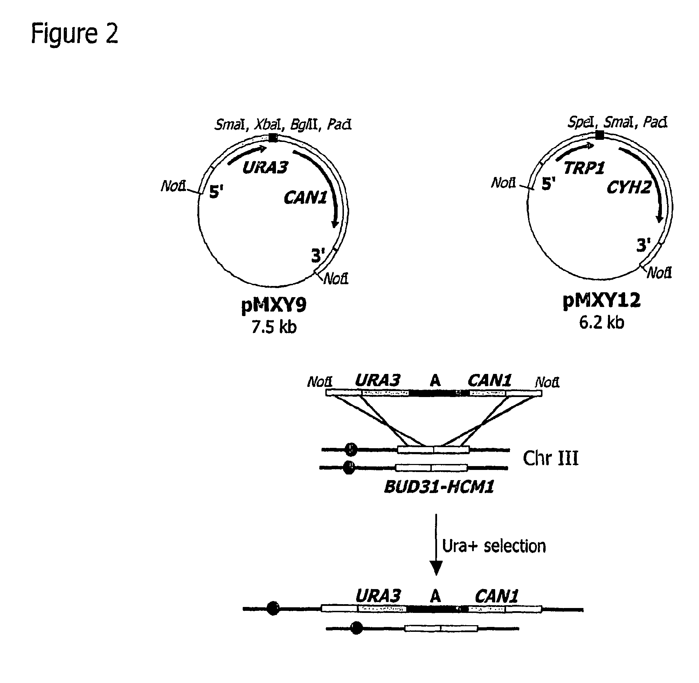

Figure 2 shows the plasmids pMXY9 and pMXY12 (above), which are vectors used for the targeting of recombination cassettes to the *BUD31-HCM1* locus on chromosome III of the yeast genome. Both plasmids bear sequences homologous to this locus (indicated as 5' and 3'), which flank the *URA3* and *CAN1* markers (pMXY9) or *TRP1* and *CYH2* (pMXY12) markers. A short sequence bearing restriction sites that allow for cloning recombination substrates is located between each pair of marker sequences. Below, integration of recombination cassettes into the *BUD31-HCM1* locus. A pMXY9 derivative bearing recombination substrate A is digested with *Not*I to liberate the recombination cassette flanked by 5' and 3' targeting sequences and digestion products are transformed into MXY47 cells. Ura+ derivatives that contain a correctly targeted insert are identified for subsequent use in constructing strains heterozygous for recombination cassettes. Recombination cassettes bearing the *TRP1* and *CYH2* markers are similarly constructed in pMXY12 and transformed into MXY47, followed by selection for tryptophan prototrophy.

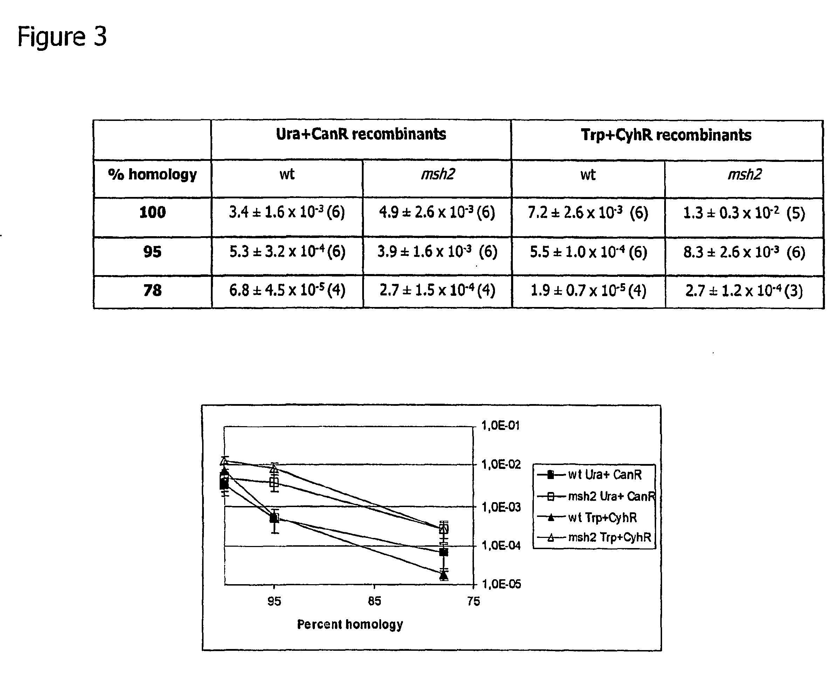

Figure 3 shows the frequency of recombination between Oxa genes as a function of sequence identity in wild type and *msh2* strains. Above, the mean ± standard deviation of (n) independent experiments is provided. Below, graphical representation of these data. The following strains were used: MXY60, MXY62, MXY64, MXY66, MXY99, and MXY102.

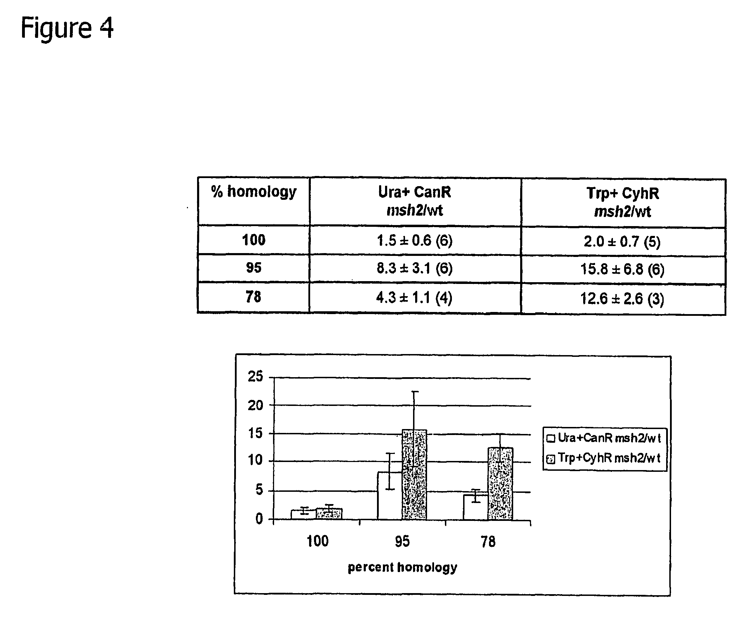

Figure 4 shows the *msh2* hyper-recombination effect. A *msh2*/wt ratio was calculated for each independent experiment (total number = n) for pairs of strains with the given percent of shared Oxa homology and for each selection condition, and the the mean ± standard deviation of these summed values are shown. The data are represented graphically below. The following pairs of strains were used: MXY60 and MXY62, MXY64 and MXY66, MXY99 and MXY102.

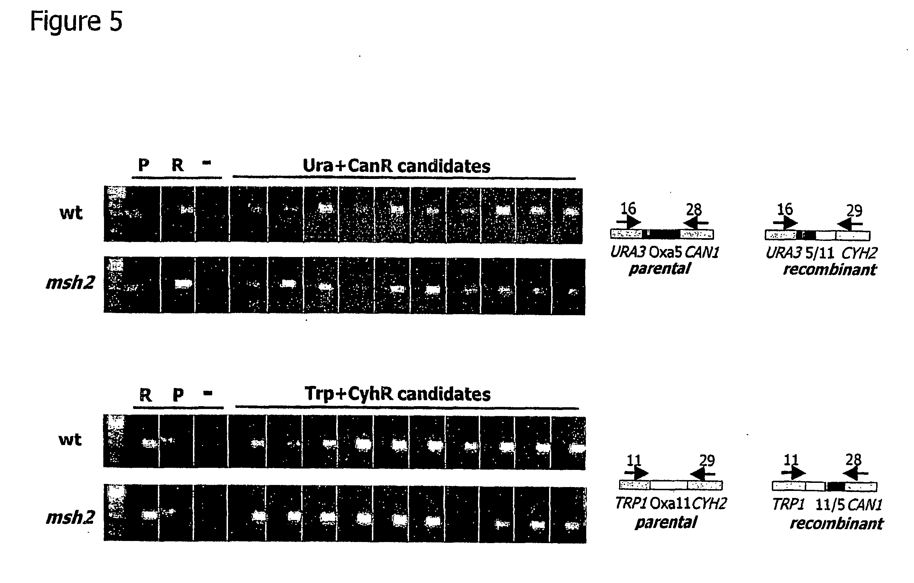

Figure 5 shows a PCR analysis of recombination between Oxa sequences sharing 78% homology. Spore colonies were derived from wild type (MXY99) and *msh2* (MXY102) diploids by selection on medium lacking uracil and containing canavanine, or on medium lacking tryptophan and containing cycloheximide. Colony PCR was performed on selected spore colonies that exhibited phenotypes consistent with those expected for crossover recombinants. Above, two reactions were carried out for each wild type and *msh2* Ura+CanR candidate, one with a parental-specific primer pair (KNS16 + KNS28, products shown in the first of each pair of lanes for each candidate), and the other with a recombinant-specific primer pair (KNS16 + KNS29, second lane). Below, similar reactions were carried out for each wild type and *msh2* Trp+CyhR candidate, one with a parental-specific primer pair (KNS11 + KNS29, first lane), and the other with a recombinant-specific primer pair (KNS11 + KNS28, second lane). Control reactions were carried out on appropriate genomic DNA templates containing known configurations of flanking marker sequences, either parental (P) or recombinant ( R). (-) no DNA control.

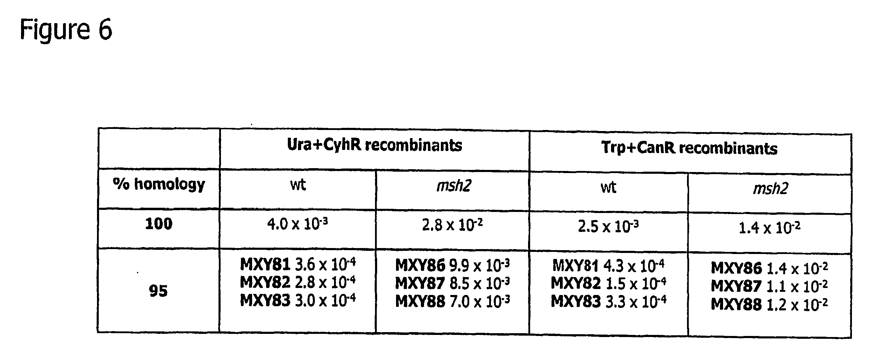

Figure 6 shows the frequencies of recombination for second-round recombination. Wild type and *msh2* haploids obtained after a first round of recombination with MXY64 and MXY66 were mated to produce wild type (MXY81, MXY82 and MXY83) and *msh2* (MXY86, MXY87, and MXY88) diploids with mosaic Oxa7-Oxa11 recombination cassettes. Wild type (MXY90) and *msh2* (MXY92) diploids homozygous for the Oxa11 recombination substrate were also constructed from recombinant progeny of MXY60 and MXY62. All diploids were sporulated and spores were plated on media to select for Ura+CanR and Trp+CyhR recombinants.

The sequence listing comprises the following sequences:
SEQ ID No. 1 and 2 show the sequences of the primers MSH2UP and MSH2DN, respectively, for the amplification of *MSH2.*
SEQ ID No. 3 to SEQ ID No. 6 show the sequences of the primers MSH2A1, MSH2A2, MSH2A3 and MSH2A4, respectively, which are *MSH2*-specific analytical primers.
SEQ ID No. 7 and SEQ ID No. 8 show the sequences of the primers K2KANMX and K3KANMX, respectively, which are *KanMX*-specific analytical primers.
SEQ ID No. 9 and SEQ ID No. 10 show the sequences of the primers LEU2UP and LEU2DN, respectively, which are used for the amplification of *LEU2.*
SEQ ID No. 11 and SEQ ID No. 12 show the sequences of the primers HIS3UP and HIS3DN, respectively, which are used for the amplification of *HIS3.*
SEQ ID No. 13 and SEQ ID No. 14 show the sequences of the primers KNS1 and KNS2, respectively, which are used for the amplification of the 3' targeting sequence.
SEQ ID No. 15 to SEQ ID No. 17 show the sequences of the primers KNS3, KNS4 and KNS6, respectively, which are used for the amplification of a 5' targeting sequence.
SEQ ID No. 18 and SEQ ID No. 19 show the sequences of the primers KNS7 and KNS8, respectively, which are used for the amplification of Oxa7.
SEQ ID No. 20 and SEQ ID No. 21 show the sequences of the primers KNS9 and KNS10, respectively, which are used for the amplification of Oxa11.
SEQ ID No. 22 shows the sequence of the primer KNS12, which is a *BUD31* downstream analytical primer.
SEQ ID No. 23 shows the sequence of the primer KNS13, which is a *BUD31* upstream analytical primer.
SEQ ID No. 24 shows the sequence of the primer KNS14, which is a *TRP1*-specific analytical primer.
SEQ ID No. 25 shows the sequence of the primer KNS15, which is a URA3-specific analytical primer.
SEQ ID No. 26 and SEQ ID No. 27 show the sequences of the primers KNS17 and KNS18, respectively, which are used for the amplification of *CYH2.*
SEQ ID No. 28 shows the sequence of the primer KNS30, which is a *TRP1*-specific forward primer used as sequencing primer.
SEQ ID No. 29 shows the sequence of the primer KNS31, which is a *CAN1*-specific reverse primer used as sequencing primer.
SEQ ID No. 30 shows the sequence of the primer KNS33, which is a *CYH2*-specific reverse primer used as sequencing primer.
SEQ ID No. 31 and SEQ ID No. 32 show the sequences of the primers KNS36 and KNS37, respectively, which are used for the amplification of Oxa5.
SEQ ID No. 33 shows the sequence of the primer KNS38, which is a *URA3*-specific forward primer used as sequencing primer.

### Example - Generation of mosaic genes in Saccharomyces cerevisiae mismatch repair mutants

### 1. Materials and methods

### 1.1 Media

Standard rich medium YPD (Bio101) was used for routine growth, and synthetic dropout media (Bio101) were used to monitor genetic markers and for selection of recombinants. For sporulation, cells were precultured overnight in SPS (50 mM potassium phthalate, pH 5.0, 0.5% yeast extract (Difco), 1% Bactopeptone (Difco), 0.17% yeast nitrogen base, 1% potassium acetate, 0.5% ammonium sulfate) plus required nutritional supplements, washed, resuspended in 1 % potassium acetate plus supplements and incubated with shaking for two days. All manipulations were carried out at 30°C. For tetrad analysis, asci were digested with *Helix pomatia* B-glucuronidase (Sigma) and dissected using a Nikon Eclipse E400 microscope fitted with a TDM400 micromanipulator (Micro Video Instruments, Inc.). Other genetic methods were conducted as described by Ausubel et al. Current Protocols in Molecular Biology (1998), John Wiley and Sons, Inc., New York. All yeast transformations were performed using the LiAc method according to Agatep et al., Technical Tips On-line (http://tto.trends.com).

### 1.2. Yeast strains

All yeast strains used or created in this study are listed in Table 1 and Table 2. All yeast strains are isogenic derivatives of the readily sporulating W303 background. The diploid MXY47, which serves as a host for transformation with recombination cassettes, was constructed by transformation and genetic crosses as follows. The haploid D184-1B (a gift of S. Gangloff, CEA, France) was transformed with a *LEU2* fragment (obtained by preparatory PCR of the W303 strain U474 with the primer pair LEU2UP/LEU2DN which are listed in the sequence listing to yield the Leu+ haploid MXY13. The haploid D184-1C (a gift of S. Gangloff) was transformed with a *HIS3* fragment (obtained by preparatory PCR of ORD4369-25D with the primer pair HIS3UP/HIS3DN) to yield the His+ haploid MXY25. The haploids MXY18 and MXY22 are recessive cycloheximide-resistant (*cyh2R*) derivatives of D184-1B and D184-1C, respectively, selected on 10 µg/ml cycloheximide; the presence of mutations mapping to the *CYH2* locus that confer cycloheximide resistance was confirmed by sequencing (two different nucleotide alterations resulting in a change of glutamine 38 to lysine) and segregation analysis. MXY18 and MXY25 were crossed to obtain the diploid MXY29; MXY13 and MXY22 were crossed to obtain the diploid MXY33. The haploid segregants MXY29-6D and MXY33-8C were crossed to obtain MXY38, which is heterozygous for the *leu2-3, 112* and *his3-11, 15* markers and homozygous for the *cyh2R* mutation. MXY38 was transformed with the *msh2::KanMX* cassette amplified by PCR from RBT348 (a gift of R. Borts, University of Leicester) with the primers MSH2UP and MSH2DN to yield MXY47. Transformants were selected on 200 µg/ml G418 (Invitrogen) and confirmed by colony PCR (see below) with the primers MSH2A1, MSH2A2, MSH2A3 and MSH2A4 and by tetrad analysis, i.e. analysis of the four spores, to confirm marker segregation.

**Table 1. Haploid yeast strains**

| **Name** | **Genotype** | **Source or derivation** |
|---|---|---|
| D184-1B | a *ura3-1 trp1-1 can1-100 his3-11, 15 leu2-3, 112 ade2-1* | S. Gangloff |
| D184-1C | *alpha ura3-1 trp1-1 can1-100 hrs3-911,15 leu2-3,112 ade2-1* | S. Gangloff |
| U474 | *alpha ura3-1 trp1-1 can1-100hrs3-11,15 ade2-1* | S. Gangloff |
| ORD4369-25D | | |
| MXY13 | *a ura3-1 trp1-1 can1-100 his3-11,15 ade2-1* | D184-1B transformed with *LEU2* PCR product |
| MXY18 | a *ura3 -1 trp1-1 can1-100 cyh2R his3-11, 15 leu2-3, 112 ade2-1* | D184-1B cyhR derivative |
| MXY22 | *alpha ura3-1 trp1-1 can1-100 cyh2R his3-11, 15 leu2-3, 112 ade2-1* | D184-1C cyhR derivative |
| MXY25 | *alpha ura3-1 trp1-1 can1-100 leu2-3, 112 ade2-1* | D184-1C transformed with *HIS3* PCR product |
| MXY29-6D | *alpha ura3-1 trp1-1 can1-100 cyh2R leu2-3, 112 ade2-1* | MXY29 segregant |
| MXY33-8C | *a ura3-1 trp1-1 can1-100 cyh2R his3-11,15 ade2-1* | MXY33 segregant |
| MXY50-3D | *alpha msh2::KanMX ura3-1 trp1-1 cyh2R can1-00 leu2-3, 112 ade2-1 BUD31::URA3-CAN1* | MXY50 segregant |
| MXY50-7D | *alpha ura3-1 trp1-1 cyh2R can1-100 his3-11,15 ade2-1 BUD31::URA3-CAN1* | MXY50 segregant |
| MXY51-2B | *alpha msh2::KanMX ura3-1 trp1-1 cyh2R can1-100 his3-11, 15 ade2-1 BUD31::URA3-Oxa7-CAN1* | MXY51 segregant |
| MXY51-10C | *alpha ura3-1 trp1-1 cyh2R can1-100 leu2-3,112 ade2-1 BUD31::URA3-Oxa7-CAN1* | MXY51 segregant |
| MXY52-2A | *alpha msh2:KanMX ura3-1 trp1-1 cyh2R can1-100 his3-11, 15 ade2-1 BUD31::URA3-Oxa11-CAN1* | MXY52 segregant |
| MXY52-7D | *alpha ura3-1 trp1-1 cyh2R can1-100 leu2-3,112 ade2-1 BUD31::URA3-Oxa11-CAN1* | MXY52 segregant |
| MXY53-11C | *a ura3-1 trp1-1 cyh2R can1-100 leu2-3,112 ade2-1 BUD31::TRP1-CYH2* | MXY53 segregant |
| MXY53-11 D | *a msh2::KanMX ura3-1 trp1-1 can1-100 cyh2R his3-11,15 ade2-1 BUD31::TRP1-CYH2* | MXY53 segregant |
| MXY55-1C | *a msh2::KanMX ura3-1 trp1-1 can1-100 cyh2R his3-11,15 ade2-1 BUD31::TRP1-Oxa11-CYH2* | MXY55 segregant |
| MXY55-2B | *a ura3-1 trp1-1 can1-100 cyh2R his3-11,15 ade2-1 BUD31::TRP1-Oxa11-CYH2* | MXY55 segregant |
| MXY55-13D | *a msh2::KanMX ura3-1 trp1-1 can1-100 cyh2R leu2-3,112 ade2-1 BUD31::TRP1-Oxa11-CYH2* | MXY55 segregant |
| | | |
| | | |
| MXY79-3B | *alpha ura3-1 trp1-1 can1-100 cyh2R his3-11,15 leu2-3,112 ade2-1 BUD31::URA3-Oxa5-CAN1* | MXY79 segregant |
| MXY79-9A | *alpha msh2::Kan MX ura3-1 trp1-1 can1-100 cyh2R leu 2-3,112 ade2-1 BUD31::URA3-Oxa5-CAN1* | MXY79 segregant |
| RBT348 | *alpha msh2::KanMX ura3 cyhR met13-4 lys2-d* | R. Borts |

**Table 2. Diploid yeast strains.**

| **Name** | **Genotype** | **Source or derivation** |
|---|---|---|
| MXY29 | *a*/*alpha ura3-1*/*" trp1-1*/*" cyh2R*/*CYH2 can1-100*/*" his3-11,15*/*HIS3 leu2-3, 112*/*" ade2-1*/*"* | MXY18 x MXY25 |
| MXY33 | *a*/*alpha ura3-1*/*" trp1-1*/*" cyh2R*/*CYH2 can1-100*/*" his3-11,15*/*" leu2-3,112*/*LEU2 ade2-1*/*"* | MXY22 x MXY13 |
| MXY38 | *a*/*alpha ura3-1*/*" trp1-1*/*" cyh2R*/*" can1-100*/*" his3-11,15*/*HIS3 leu2-3,112*/*LEU2 ade2-1*/*"* | MXY33-8C x MXY29-6D |
| MXY47 | *a*/*alpha msh2::KanMX*/*MSH2 ura3-1*/*" trp1-1*/*" cyh2R*/*" can1-100*/*" his3-11,15*/*HIS3 leu2-3, 112*/*LEU2 ade2-1*/*"* | MXY38 transformed with msh2::KanMX PCR product |
| MXY50 | *a*/*alpha msh2::KanMX*/*MSH2 ura3-1*/*" trp1-1*/*" cyh2R*/*" can1-100*/*" his3-11,15*/*HIS3 leu2-3, 112*/*LEU2 ade2-1*/*" BUD31::URA3-CAN1*/*BUD31* | MXY47 transformed with *Not*1-digested pMXY9 |
| MXY51 | *a*/*alpha msh2::KanMX*/*MSH2 ura3-1*/*" trp1-1*/*" cyh2R*/*" can1-100*/*" his3-11,15*/*HIS3 leu2-3, 112*/*LEU2 ade2-1*/*" BUD31::URA3-Oxa7-CAN1*/*BUD31* | MXY47 transformed with *Not*1-digested pMXY13 |
| MXY52 | *a*/*alpha msh2::KanMX*/*MSH2 ura3*/*" trp1-1*/*" cyh2R*/*" can1-100*/*" his3-11,15*/*HIS3 leu2-3, 112*/*LEU2 ade2-1*/*" BUD31::URA3-Oxa11-CAN1*/*BUD31* | MXY47 transformed with *Not*1-digested pMXY14 |
| MXY53 | *a*/*alpha msh2::KanMX*/*MSH2 ura3-1*/*" trp1-1*/*" cyh2R*/*" can1-100*/*" his3-11,15*/*HIS3 leu2-3, 112*/*LEU2 ade2-1*/*" BUD31:: TRP1-CYH2*/*BUD31* | MXY47 transformed with *Not*1-digested pMXY12 |
| MXY55 | *a*/*alpha msh2::KanMX*/*" MSH2 ura3-1*/*" trp1-1*/*" cyh2R*/*"can1-100*/*" his3-11,15*/*HIS3* /*eu2-3, 112*/*LEU2 ade2-1*/*" BUD31::TRP1-Oxa11-CYH2*/*BUD31* | MXY47 transformed with *Not*1-digested pMXY22 |
| MXY57 | *a*/*alpha msh2::KanMX*/*" ura3-1*/*" trp1-1*/*" cyh2R*/*" can1-100*/*" his3-11,15*/*HIS3 leu2-3,112*/*LEU2 ade2-1*/*" BUD31::TRP1-CYH2*/*BUD31::URA3-CAN1* | MXY50-3D x MXY53-11D |
| MXY59 | *a*/*alpha ura3-1*/*" trp1-1*/*" cyh2R*/*" can1-100*/*" his3-11,15*/*HIS3 leu2-3,112*/*LEU2 ade2-1*/*" BUD31::TRP1-CYH2*/ *BUD31::URA3-CAN1* | MXY50-7D x MXY53-11C |
| MXY60 | *a*/*alpha ura3-1*/*" trp1-1*/*" cyh2R*/*" can1-100*/*" his3-11,15*/*HIS3 leu2-3,112*/*LEU2 ade2-1*/*" BUD31::TRP1-Oxa11-CYH2*/*BUD31::URA3-Oxa11-CAN1* | MXY52-7D x MXY55-2B |
| MXY62 | *a*/*alpha msh2::KanMX*/*" ura3-1*/*" trp1-1*/*" cyh2R*/*" can1-100*/*" his3-11,15*/*HIS3 leu2-3,112*/*LEU2 ade2-1*/*" BUD31::TRP1-Oxa11-CYH2*/*BUD31::URA3-Oxa11-CAN1* | MXY52-2A x MXY55-1C |
| MXY64 | *a*/*alpha ura3-1*/*" trp1-1*/*" cyh2R*/*" can1-100*/*" his3-11,15*/*HIS3 leu2-3,112*/*LEU2 ade2-1*/*" BUD31::URA3-Oxa7-CAN1*/*BUD31::TRP1-Oxa11-CYH2* | MXY51-10C x MXY55-2B |
| MXY66 | *a*/*alpha msh2::KanMX*/*" ura3-1*/*" trp1-1*/*" cyh2R*/*" can1-100*/*" his3-11,15*/*HIS3 leu2-3,112*/*LEU2 ade2-1*/*" BUD31::TRP1-Oxa11-CYH2*/*BUD31::URA3-Oxa7-CAN1* | MXY51-2B x MXY55-13D |
| | | |
| MXY79 | *a*/*alpha msh2::KanMX*/*MSH2 ura3-1*/*" trp1-1*/*" cyh2R*/*" can1-100*/*" his3-11,15*/*HIS3 leu2-3,112*/*LEU2 ade2-1*/*" BUD31::URA3-Oxa5-CAN1*/*BUD31* | MXY47 transformed with *Not*1-digested pMXY24 |
| | | |
| | | |
| MXY99 | *a*/*alpha ura3-1*/*" trp1-1*/*" cyh2R*/*" can1-100*/*" his3-11,15*/*" leu2-3,112*/*LEU2 ade2-* 1/*" BUD31::URA3-Oxa5-CAN1*/*BUD31::TRP1-Oxa11-CAN1* | MXY79-3B x MXY55-2B |
| MXY102 | *a*/*alpha msh2::KanMX*/*" ura3-1*/*" trp1-1*/*" cyh2R*/*" can1-100*/*can1 his3-11,15*/*HIS3 leu2-3,112*/*LEU2 ade2-1*/*" BUD31::URA3-Oxa5-CAN1*/*BUD31::TRP1-Oxa11-CAN1* | MXY79-9A x MXY55-1C |

### 1.3 Plasmid construction

The bacterial strains XL1-Blue MRF'(Δ*mcrA)183(mcrCB-hsdSMR-mrr)173 endA1 supE44 thi-1 recA1 gyrA96 relA1 lac [F' proAB lac*/*^{q}Z*Δ*M15 Tn10 (Tet^{r})]*) and JM110 *(rpsL [Str^{r}] thr leu thi-1 lacY galK galt ara tonA tsx dam dcm supE44* Δ*[lac-proAB] [F' traD36 proAB lacl^{q}Z*Δ*M15]*) were used as hosts for cloning. Standard methods were used for plasmid construction (Ausubel et al.). All plasmids used or created in this study are listed in Table 3. Restriction enzymes, T4 DNA ligase and other enzymes used in cloning were purchased from New England BioLabs. DNA fragments and plasmids were purified using kits supplied by Qiagen and Macherey-Nagel.

Upstream ("5' target") sequences corresponding to the *BUD31* locus were amplified by preparatory PCR from W303 genomic DNA with the primer pair KNS3/KNS4 and cloned as a *Kpn*1/*Xho*1 fragment into *Kpn*1/*Xho*1-digested pKSII(+) (Stratagene) to create pMXY1; downstream ("3' target") targeting sequences were similarly amplified with the primer pair KNS1/KNS2 and cloned as a *Xba*1/*Not*1 fragment into *Xba*1/*Not*1-digested pKSII(+) (Stratagene) to create pMXY2. The *TRP1* marker was excised from pJH53 (a gift of R. Borts) as a *Bgl*II/*Eco*RI fragment and ligated to *Bam*HI/*Eco*RI-digested pMXY1 to create pMXY3, and the *URA3* marker was excised from *Xho*I/*Hin*DIII-digested pRED316 (a gift of R. Borts) and ligated to *Xho*I/*Hin*DIII-digested pMXY1 to create pMXY4. The *CAN1* marker was isolated from pRED316 as a *Sma*1 fragment and ligated to *Hpa*1-digested pMXY2 to create pMXY5. The 5' targeting sequences in pMXY3 and pMXY4 were replaced with sequences reamplified from genomic DNA with the primer pair KNS4/KNS6 and ligated as *Kpn*1/*Xho*1 fragments into the respective *Kpn*1/*Xho*1-digested plasmids to produce pMXY7 and pMXY6. This step was undertaken to correct the absence from the primer KNS3 of restriction sites required in later phases of cloning. The *Kpn*I*-Sma*I fragment of pMXY6 containing the 5' target and the *URA3* marker were ligated to *Kpn*I/*Sma*I-digested pMXY5 to produce the *URA3-CAN1* recombination cassette vector pMXY9. The *Kpn*I/*Spe*I fragment of pMXY7 containing the 5' target and *TRP1* marker were ligated to *Kpn*I/*Spe*I-digested pMXY2 to produce pMXY11. Finally, the *CYH2* marker was amplified by preparatory PCR from W303 genomic DNA with the primer pair KNS17/KNS18, digested with *Bam*HI and *Pvu*I, and ligated to *Bgl*II/*Pac*I-digested pMXY11 to create the *TRP1-CYH2* recombination cassette vector pMXY12. All plasmid constructs were introduced into bacterial hosts by electroporation and verified by restriction analysis, and pMXY9 and pMXY12 were further verified by sequencing of all cloning junctions.

B-lactamase recombination substrates were amplified by preparatory PCR from host plasmids (provided by W. Schoenfeld) using the primer pairs KNS36/KNS37 for Oxa5 (accession X58272), KNS7/KNS8 for Oxa7 (accession X75562), and KNS9/KNS10 for Oxa11 (accession Z22590). Oxa7 and Oxa11 PCR products were digested with *Pac*I and ligated to *Sma*I/*Pac*I-digested pMXY9 to create pMXY13 and pMXY14, respectively, and the Oxa11 PCR product was also digested with Spel and *Pac*I and ligated to *Spe*I/*Pac*I-digested pMXY12 to create pMXY22. The Oxa5 PCR products were digested with *Bam*HI and *Pac*I and ligated to *Bgl*II/*Pac*I-digested pMXY9 to create pMXY24. All constructs were verified by restriction analysis.

**Table 3. Plasmids**

| **Name** | **Description or insert** | **Source** |
|---|---|---|
| pKSII (+) | Parental vector | Stratagene |
| pRED316 | *URA3* and *CAN1* source | R. Borts |
| pJH53 | *TRP1* source | R. Borts |
| pMXY1 | 5' target | This work |
| pMXY2 | 3' target | This work |
| pMXY3 | 5' target-*TRP1* | This work |
| pMXY4 | 5' target-*URA3* | This work |
| pMXY5 | *CAN1*-3' target | This work |
| pMXY6 | 5' target-*URA3* | This work |
| pMXY7 | 5' target-*TRP1* | This work |
| pMXY9 | 5' target-*URA3*-*CAN1*-3' target | This work |
| pMXY11 | 5' target-*TRP1*-3' target | This work |
| pMXY12 | 5' target-*TRP1*-*CYH2*-3' target | This work |
| pMXY13 | 5' target-*URA*3-Oxa7-*CAN1*-3' target | This work |
| pMXY14 | 5' target-*URA3*-Oxa11-*CAN1*-3' target | This work |
| pMXY22 | 5' target-*TRP1*-Oxa11-*CYH2*-3' target | This work |
| | | |
| pMXY24 | 5' target-*URA3*-Oxa5-*CAN1*-3' target | This work |

### 1.4 Recombinant selection and characterization

For first round recombination, plasmids bearing recombination cassettes were digested with *Not*1 and total digestion products were used to transform MXY47. Uracil (for pMXY9 derivatives) or tryptophan (for pMXY12 derivatives) prototrophs were selected, and targeting of one of the two chromosomal copies of the *BUD31-HCM1* locus by the introduced construct was confirmed by colony PCR using the primers KNS12/KNS13/KNS15 for *URA3-CAN1* derivatives and the primers KNS12/KNS13/KNS14 for *TRP1-CYH2* derivatives, which allow fragments from the intact and from the disrupted *BUD31-HCM1* loci to be amplified. Transformed heterozygotes were sporulated and tetrad analysis was carried out to identify wild type or *msh2* haploids bearing recombination cassettes. Appropriate haploids of opposite mating type were patched on YPD plates, allowed to grow overnight, mixed together on the same YPD plate and allowed to mate overnight. The mating plate was replica plated to - Ura-Trp medium to select for diploids, which were inoculated the following day in bulk into SPS plus supplements and cultured overnight. The preculture was spun down and washed, and the cells were resuspended in 1 % K acetate plus supplements and incubated for two days.

Sporulated cells were harvested, quantified, and in some cases dissected to confirm appropriate segregation of all markers. Asci were digested with zymolyase-20T (ICN Biomedicals) to liberate spores, the spore suspension was sonicated (Branson Model 250 Digital Sonifier), and appropriate dilutions were plated on YPD to determine cell viability, on uracil dropout media containing 60 ug/ml canavanine (Sigma) to select Ura+CanR recombinants, and on tryptophan dropout media containing 3 ug/ml cycloheximide (Sigma) to select Trp+CyhR recombinants. Spore colonies arising on each medium were counted and subjected to phenotypic and molecular tests to determine whether they represented true recombinants. For phenotypic analysis, a representative number of candidate recombinants was restreaked to the same medium used for selection and then replica plated to -Ura, -Trp, cycloheximide (10 µg/ml), canavanine (60 µg/ml), and mating type tester plates.

Spores were also plated on - Ura-Trp media to determine the frequency of diploids for each spore preparation, which in all cases was lower than 4% of total viable cells. For molecular analysis, total genomic DNA was subjected to analytical PCR (see below) using appropriate primer pairs that specifically amplify parental or recombinant fragments. The frequencies of recombination for a given selection are expressed as the frequency of viable cells on a given selection medium, corrected for the presence of non-recombinants exhibiting a false positive phenotype. In most cases, such false positives arose by mutational inactivation of the *CAN1* or *CYH2* marker, as suggested by analytical PCR.

For second round recombination, appropriate recombinants derived from the first round of recombination were mated and Ura+Trp+ diploids were selected. The same sporulation procedure as for first round recombination was followed, except that spores were plated on YPD, on uracil dropout media containing cycloheximide to select Ura+CyhR recombinants, and on tryptophan dropout media containing canavanine to select Trp+CanR recombinants. Candidate recombinants were similarly subjected to phenotypic and molecular analysis.

### 1.5 Molecular methods

Genomic DNA used as a template for preparatory or analytical PCR was prepared from overnight YPD cultures by a standard miniprep procedure according to Ausubel et al. Preparatory PCR of fragments used in cloning or for sequencing was performed with Oxa plasmid DNA (approximately 50 pg) or yeast genomic DNA (approximately 0.5 µg) as a template in 50 µl reactions containing 2.5 U Herculase polymerase (Stratagene), 1x Herculase reaction buffer, 0.2 mM each dNTP and 100 ng each primer. Amplification was carried out as follows: 94° C 2 min; 30 cycles of 94°C 10s, 55°C 30 s, 72°C 30s; 68°C 10 min. A modified colony PCR procedure was employed to confirm integration of recombination cassettes at the *BUD31* locus (http://www.fhcrc.org/labs/hahn/methods/mol bio meth/pcr yeast c olony.html), with the following amplification conditions: 95°C 5 min; 35 cycles of 95°C 1 min, 55°C 1 min, 68°C 1 min; 72°C 10 min. Analytical PCR to characterize Oxa inserts was carried out in 100 µl reaction volumes containing approximately 0.5 µg genomic DNA prepared from candidate recombinants and control strains, 1.5 U Taq polymerase (Roche), 1 x reaction buffer, 0.2 mM each dNTP and 100 ng each primer, with the same amplification conditions as for colony PCR, except that extension was carried out at 68°C for 2 min. All amplification reactions were performed with a Mastercycler gradient 5331 (Eppendorf).

For sequence analysis of recombinant Oxa inserts, preparatory PCR was carried out with the primer pairs KNS16/KNS29 (for Ura+CanR recombinants) or KNS11/KNS28 (for Trp+CyhR), followed by purification with the Qiaquick PCR kit (Qiagen). PCR products were sequenced by Genome Express (Meylan, FR) with the primers KNS30, KNS31, KNS33 or KNS38, as appropriate. Recombinant sequences were aligned and analyzed using Clone Manager software (Sci Ed Central). Oligonucleotides used in PCR and sequencing (Table 3) were purchased from Proligo France.

### 2. Results

### 2.1 Development of a yeast meiotic homeologous recombination system

A strategy that makes use of the yeast *Saccharomyces cerevisiae* to promote *in vivo* recombination between diverged DNA sequences has been developed. Critical features of the strategy include the use of meiotic cells, in which high levels of genome-wide recombination take place, and inactivation of the mismatch repair (MMR) system, which normally restricts recombination between diverged sequences. Sequences to be recombined, i.e. the recombination substrates, are introduced into one of two vectors that also bears flanking marker sequences, so as to create recombination cassettes. The recombination cassettes are introduced into the yeast genome, at a locus on chromosome III (the *BUD31-HCM1* interval), which is in a region known to be recombinationally active in meiosis. Diploids heterozygous for recombination cassettes are sporulated, and spores are plated on media that select for cells with specific configurations of flanking markers, thereby allowing for the selection of recombinants in which a crossover involving recombination substrates has taken place (Figure 1).

Two general recombination cassette vectors were constructed, pMXY9 and pMXY12, which contain the *URA3* and *CAN1,* and the *TRP1* and *CYH2* markers, respectively, flanking restriction sites that can be used for the introduction of recombination substrates (Figure 2). The *URA3* marker confers uracil prototrophy, and the *CAN1* marker confers canavanine sensitivity. In the absence of this marker, cells are resistant to the drug. The *TRP1* marker confers tryptophan prototrophy and the *CYH2* marker confers cycloheximide sensitivity. In the absence of this marker, cells are resistant to the drug. Each of the two recombination cassettes is in turn flanked by sequences that allow targeting of the entire insert to the *BUD31-HCM1* locus by transformation of competent cells (Figure 2). A strain that serves as a primary host for transformation, MXY47, was also constructed (Table 2). This diploid is heterozygous for the *msh2::KanMX* mutation, and is phenotypically wild type with respect to MMR. It is also homozygous for the *ura3-1, trp1-1, can1-100* and *cyh2R* markers, which allows the presence of recombination cassette markers to be monitored, and heterozygous for the *his3-11,15* and *leu2-3,112* markers. MXY47 is transformed with fragments bearing recombination cassettes, primary transformants are selected as Ura+ or Trp+ prototrophs (for MXY9 and MXY12 derivatives, respectively), and targeting is confirmed by analytic PCR using primers that recognize sequences within and external to the introduced construct. Primary transformants are sporulated, and tetrads are dissected and replica plated to identify wild type or *msh2* segregants that bear the recombination cassette. Suitable haploids are mated to one another to generate *MSH2*/*MSH2* (wild type) and *msh2*/*msh2* diploids heterozygous for recombination substrates. In a first round of meiotic recombination to generate recombinants, these diploids are sporulated, and free spores are plated on media lacking uracil and containing canavanine to select for recombinants with the *URA3-CYH2* configuration of flanking markers (Ura+CanR spore colonies), or on media lacking tryptophan and containing cycloheximide to select for the *TRP1-CAN1* configuration (Trp+CyhR spore colonies). Parental diploids and non-recombinant haploid progeny cannot grow on these media. The frequency of spore colonies arising on selective media is determined, candidate recombinant spore colonies are characterized phenotypically by replica plating on test media and molecularly by PCR with appropriate primer pairs, and a sample of confirmed recombinants is selected for sequencing.

The strategy is iterative in that cells bearing recombinant inserts can be identified and subjected to further rounds of meiotic recombination to increase diversity. In a second round, Ura+CanR and Trp+CyhR haploids are mated, and the sporulation and selection process is repeated, except that new recombinants are selected on media lacking uracil and containing cycloheximide, to select for recombinants with the *URA3-CAN1* configuration of flanking markers (Ura+CyhR spore colonies), or on media lacking tryptophan and containing canavanine to select for the *TRP1-CYH2* configuration (Trp+CanR spore colonies). The strategy detailed here can also be modified to include additional markers to increase the stringency of selection. Furthermore, recombinants can also be directly selected by PCR using primers specific to flanking sequences.

### 2.2 Phenotypic selection for recombination between Oxa gene pairs of varying sequence divergence

Genes belonging to the Oxa superfamily of beta-lactamases were chosen as substrates to test the feasibility of the system for the selection of recombinants. Recombination between the following Oxa pairs was assessed in the wild type and *msh2* backgrounds: Oxa11-Oxa11, which share 100% homology throughout the 800 bp ORF; Oxa7-Oxa11, 95%; Oxa5-Oxa11, 78%. Diploids generated by crosses between appropriate haploids were induced to enter meiosis. Spores were prepared from meiotic cultures, and serial dilutions were plated on YPD to determine cell viability and on medium lacking uracil and containing canavanine (-Ura+Can) and on medium lacking tryptophan and containing cycloheximide (-Trp+Cyh) to select for recombinants.

### 2.3 Frequencies of recombination between Oxa genes of varying sequence homology

The data shown in Figure 3 demonstrate that in the wild type background, increased sequence heterology has a strong inhibitory effect on crossover recombination, and that this effect is relieved but not abolished by the *msh2* mutation. In general, the *msh2* mutation causes an increase in the frequency of recombination of about one order of magnitude above that observed for wild type strains at the two levels of divergence tested. However, inactivation of *MSH2* alone does not fully compensate for the inhibition of recombination between recombination substrates with higher degrees of heterology. For example, the frequencies of recombination for a *msh2* strain with Oxa inserts sharing 78% homology (MXY102) are at least 10-fold (Ura+CanR) and 25-fold (Trp+CyhR) below those found for a wild type strain with Oxa inserts of 100% homology (MXY60), indicating that factors other than MSH2-dependent mismatch repair prevent crossover recombination between more diverged sequences. It is noteworthy that the appearance of *msh2* recombinants at the 78% divergence level, at frequencies of roughly 2 x 10⁻⁴, indicates that recombination may be achieved between even more divergent substrates.

### 2.4 The msh2 hyper-recombination effect

The effect of *msh2* on homologous and homeologous recombination was quantified by first calculating the ratio of *msh2* to wild type recombinants for a given percent of homology for a given selection for each experiment, and then calculating the means and standard deviations of the ensemble of ratios thus determined. The data are shown in in Figure 4. The presence of the *msh2* mutation increases the frequency of homeologous recombination for sequences of 95% and 78% homology, and there is a less pronounced but still quantifiable enhancement of recombination between 100% identical sequences. Furthermore, the extent of the *msh2* enhancement of homeologous recombination differs for the two selections: for strains with homeologous Oxa inserts, inactivation of *MSH2* increases the frequency of Trp+CyhR recombinants to a greater extent than it increases the frequency of Ura+CanR recombinants. In principle, the frequencies of both types of recombinants (Ura+CanR and Trp+CyhR) should be equivalent, but these numbers indicate that there are biases in the system that are provoked or enhanced by the *msh2* mutation, in conjunction with variations in the extent of sequence divergence. Experiments to test the relative influences of inserts and flanking marker sequences on the types of recombinants obtained indicate that this bias is a property of the flanking markers but the influence of the recombination substrates in directing the outcomes of meiotic recombination events cannot yet be accounted for (data not shown).

### 2.5 PCR analysis of selected recombinants

An example of PCR analysis, as applied to Ura+CanR and Trp+CyhR spore colonies derived from wild type and *msh2* diploids containing Oxa genes of 22% divergence (MXY99 and MXY102, respectively) is shown in Figure 5. For each strain, ten spore colonies that exhibited each recombinant phenotype were analyzed. Extracts from each colony were used as templates for amplification with primer pairs that specifically amplify parental molecules and with primer pairs that specifically amplify recombinant molecules. In every case(s), only the predicted recombinant insert was amplified, indicating that the selected spore colonies contained sequences produced by recombination between the parental Oxa recombination substrates. These results also demonstrate that recombinant molecules can be directly recovered from sporulated cultures, even without the imposition of a genetic selection step. Here, the primer recognition sites located in the *UPA3, CAN1, TRP1* and *CYH2* genes represent molecular marker sequences that flank each recombination substrate.

### 2.6 Sequence analysis of first round meiotic homeologous recombinants: 5% and 22% divergence

Oxa7-Oxa11 meiotic recombinants derived from wild type and *msh2* diploids (MXY64 and MXY66, respectively), which contain recombination substrates sharing 95% homology, that satisfied phenotypic and molecular (PCR) tests were subjected to sequence analysis. Recombinant fragments were amplified with primers specific to flanking markers and sequenced using primers close to the translational start and stop sites. Overall, 55 recombinant sequences derived from haploid progeny of Oxa7-Oxa 11 diploids were analyzed: 14 Ura+CanR and 13 Trp+CyhR recombinants from MXY64, and 14 Ura+CanR and 14 Trp+CyhR recombinants from MXY66. The sequenced sample size allows several observations to be made. 1) For both wild type and *msh2* recombinants, the position at which the crossover took place ranged throughout the full coding region, with no apparent preference for a specific interval. Crossovers that occurred in the 5' region were as likely as those in the 3' region. Also, for a given strain, there was no apparent difference in the distributions of crossover sites for spore colonies obtained by -Ura+Can or by -Trp+Cyh selection. 2) The length of uninterrupted homology in the crossover interval was also unimportant: crossovers were detected between two closely spaced polymorphisms (positions 543-552 for MXY66 Trp+CyhR #7, #8, and #13, where position 1 represents the adenosine residue of the ATG translational start site) as well as between the two most widely spaced polymorphisms (positions 163-265, eg MXY66 Trp+CyhR #15). 3) The recombinant Oxa inserts isolated from both wild type and *msh2* backgrounds contained full-length recombinant sequences potentially capable of encoding new, functional Oxa proteins. That is, all crossovers occurred in such a manner as to preserve an intact ORF, without a net insertion or deletion of nucleotides in the crossover interval or in any other interval. 4) Although the structures of most recombinant sequences are consistent with a simple crossover between the two Oxa sequences in local regions of homology, several recombinants isolated in the *msh2* background exhibited greater complexity. Sequences derived from four recombinants (MXY66 Ura+CanR #16 and #31, and MXY66 Trp+CyhR #5 and #9) exhibited a higher degree of mosaicism, as if they were produced by more than one crossover event. Indeed, analysis of two of these recombinants was complicated because inspection of electropherograms revealed the presence of two overlapping peaks at multiple sites within the sequenced region, each site corresponding to an Oxa 7-Oxa11 polymorphism. This observation indicates that the population of molecules that was sequenced was heterogeneous, for which the most likely explanation is the presence of unrepaired or partially repaired heteroduplex DNA present in *msh2* recombinant spores. This interpretation is consistent with the known increased frequency of post-meiotic segregation (PMS) caused by the *msh2* mutation. In these two cases, MXY66 Ura+CanR #16 and #31, one or more repaired sites was flanked by stretches of unrepaired heteroduplex, consistent with the unmasking of a short patch mismatch repair activity in the *msh2* background, as suggested by Coïc, Gluck and Fabre (EMBO J. 19:3408). Several other cases of PMS unassociated with short-patch mismatch repair were also observed for *msh2* recombinant sequences, indicating that this alternative mismatch repair system may not be highly efficient at correcting mismatches in heteroduplex DNA. Judging from sequence electropherograms, the extent of uncorrected heteroduplex varied, from a short region of about 50 nt to a region almost covering the entire ORF. No evidence for PMS or short-patch mismatch repair was found for wild type recombinant sequences. Overall, these findings suggest that the extent of diversity created is greater in *msh2* meiosis than in wild type meiosis.
Meiotic recombinants were also derived from wild type and *msh2* diploids that contain recombination substrates sharing 78% homology, (MXY99 and MXY102, respectively). In total, 24 recombinant sequences derived from recombinant progeny of Oxa5-Oxa 11 diploids were analyzed: five Ura+CanR and three Trp+CyhR recombinants from MXY99, and nine Ura+CanR and seven Trp+CyhR recombinants from MXY102. Inspection of these sequences suggests several trends. 1) Recombinant Oxa sequences obtained In both wild type and *msh2* strains by selection on -Trp+CyhR exhibited crossovers at different positions throughout the ORF, with perhaps a slight tendency towards the middle 250 bp region (nt 333-nt 573) of overall shared homology. In contrast, recombinants obtained in both wild type and *msh2* strains by selection on -Ura+CanR exhibited a pronounced bias in the positions of crossovers: in 3 of 5 wild type and 8 of 9 msh2 sequences, crossovers occurred within the last 80 nt of the region of shared homology, ie, the last 10% of the ORF. 2) The intervals of absolute homology in which crossovers were identified ranged from 11 to 20 nt for the -Trp+Cyh selection, indicating a preference for these relatively larger regions of sequence identity. In contrast, crossover intervals were shorter for the -Ura+Can selection, ranging from 3 to 17 nt (13/14 of these involved intervals 13 nt and shorter). 3) As for recombination involving sequences sharing 95% homology, the new sequences obtained also consisted of intact ORFs and potentially encode novel Oxa proteins. 4) No cases of PMS, as judged by inspection of electropherograms, were found for wild type recombinants, but very short patches of unrepaired heteroduplex were found for a few *msh2* recombinants, including 3 of the 7 Trp+CyhR recombinants. These regions included at most 67 nt, shorter than some of the tracts observed for Oxa7-Oxa11 recombinants. In sum, these observations indicate that recombinant sequences can be selected from input recombination substrates varying by at least 22%, and that these sequences encode novel proteins.

### 2.7 Sequence analysis of Oxa7-Oxa11 second-round recombinants

The ability of the yeast system to increase sequence diversity in an iterative manner was tested by constructing diploids from Oxa7-Oxa11 recombinant haploids generated in a first round of meiosis and subjecting these new diploids to a second round of meiosis. Among the sequenced Ura+CanR and Trp+CyhR progeny of MXY64 and MXY66, pairs of appropriate recombinants with crossovers in the same interval were selected to construct new diploids in which the overall level of sequence homology was again 95%. Three wild type (MXY81, MXY82 and MXY83) and three *msh2* (MXY86, MXY87 and MXY88) diploids were created. Control wild type and *msh2* diploids containing only Oxa11 sequence inserts were also constructed from appropriate recombinant progeny of MXY60 and MXY62, yielding MXY90 and MXY92. These diploids were sporulated and spores were plated on medium lacking uracil and containing cycloheximide (-Ura+Cyh) and on medium lacking tryptophan and containing canavanine (-Trp+Can) to select for second-round recombinants. As shown in Figure 6, the frequencies of -Ura+CyhR and Trp+CanR spore colonies observed for all of these strains is consistent with the anti-recombination effect of the *MSH2* gene. Both types of colonies were found among progeny of the wild type homozygote MXY90 at frequencies above 10⁻³, whereas these frequencies were decreased 5- to 10-fold among progeny of wild type diploids with Oxa insert heterology (MXY81, MXY82 and MXY83). Inactivation of the *MSH2* gene in diploids with diverged Oxa inserts (MXY 86, MXY 87, and MXY 88) led to a 2 to 6-fold increase in the frequency of Ura+CyhR and Trp+CanR spore colonies, similar to the levels seen for a *msh2* diploid bearing identical Oxa inserts (MXY92). Although the media used differ from those used for selection of first-round recombinants, the frequencies at which wild type and *msh2* second-round recombinants were selected are comparable to those for first-round recombinants.

Ura+CyhR and Trp+CanR spore colonies in both the wild type (MXY81 and MXY83) and msh2 (MXY86) backgrounds were selected for sequencing. In all, 14 wild type and 7 msh2 Oxa inserts were sequenced. In most cases, a crossover occurred in a novel interval during second round recombination, again without apparent bias with respect to position or interval size: crossovers involving different intervals were found throughout the Oxa ORF and they occurred in intervals as large as 101 nt and as small as 5 nt. In one case (a MXY83 Trp+CyhR haploid), a second round crossover occurred in the first round crossover interval, thereby restoring a full Oxa11 sequence. Recombinants recovered from msh2 diploids were more diverse than those recovered from wild type strains. For the msh2 diploid MXY86 several spore colonies exhibiting extensive PMS and sequence mosaicism were observed, consistent with the formation of long tracts of heteroduplex in the recombinational intermediate. Furthermore, some mismatches were repaired in the heteroduplex tract, again consistent with a short-patch mismatch repair activity. In sum, second-round recombination in the msh2 background is as efficient as first-round recombination, both qualitatively, with respect to generating sequence diversity (eg., crossover interval distribution and incidence of PMS), and quantitatively, with respect to increasing the overall frequency of homeologous (5% divergence) recombination.

## Claims

1. Process for generating and detecting recombinant DNA sequences in Saccharomyces cerevisiae comprising the steps of:
a) generating first diploid S. cerevisiae cells bearing in a defined locus of their genome a first recombination cassette (1) comprising a first DNA sequence to be recombined (A), which is flanked by at least a first and a second marker sequence, and in an allelic position a second recombination cassette (2) comprising a second DNA sequence to be recombined (B), which is flanked by at least a third and a fourth marker sequence,
b) inducing the sporulation of the first diploid cells obtained in a)
and
c) isolating haploid cells containing recombination cassettes (3) in which first recombined DNA sequences (A/B) are flanked by at least the first and fourth marker sequences, and haploid cells containing recombination cassettes (4) in which second recombined DNA sequences (B/A) are flanked by at least the second and the third marker sequences.

2. Process according to claim 1, comprising further the steps of:
a) generating second diploid cells by mating haploid cells containing the first recombined DNA sequences (A/B) obtained in 1c) with haploid cells containing the second recombined DNA sequences (B/A) obtained in 1c),
b) inducing the sporulation of the second diploid cells obtained in a) b) inducing the sporulation of the second diploid cells obtained in a) and
c) isolating haploid cells containing recombination cassettes in which third recombined DNA sequences are flanked by at least the first and the second marker sequences, and haploid cells containing fourth recombination cassettes in which fourth recombined DNA sequences are flanked by at least the third and the fourth marker sequences.

3. Process according to claim 1 or 2, wherein further recombined DNA sequences are generated by subjecting the haploid cells obtained in 2c) at least once to another cycle of mating with other haploid cells, inducing the sporulation of the diploid cells obtained and isolating haploid cells with recombined DNA sequences on the basis of the molecular linkage between two marker sequences.

4. Process according to any one of claims 1 to 3, wherein the first diploid cell is generated by simultaneously or sequentially transforming a diploid S. cerevisiae cell with a DNA molecule containing the first recombination cassette (1) and a DNA molecule containing the second recombination cassette (2) and allowing the integration of the two recombination cassettes into allelic positions of the S. cerevisiae genome.

5. Process according to claim 4, wherein the DNA molecule comprising the first or the second recombination cassettes (1, 2) is a yeast artificial chromosome (YAC).

6. Process according to claim 4, wherein the DNA molecule comprising the first or the second recombination cassette is a cloning vehicle, whereby the respective two marker sequences are flanked by targeting sequences which are homologous to a defined locus of the S. cerevisiae genome.

7. Process according to any one of claims 1 to 3, wherein the first diploid cell is generated by fusing a haploid S. cerevisiae cell bearing in a locus of its genome the first recombination cassette with a haploid S. cerevisiae cell bearing in an allelic position the second recombination cassette.

8. Process according to any one of claims 1 to 3, wherein the first diploid cell is generated by mating a haploid S. cerevisiae cell bearing in a locus of its genome the first recombination cassette with a haploid S. cerevisiae cell bearing in an allelic position the second recombination cassette.

9. Process according to claim 7 or 8, wherein haploid cells bearing the first or second recombination cassette are generated by:
a) inserting the first DNA sequence to be recombined between the first and the second marker sequences located adjacently on a first cloning vehicle and inserting the second DNA sequence to be recombined between the third and the fourth marker sequences located adjacently on a second cloning vehicle, whereby the respective two marker sequences are flanked by targeting sequences which are homologous to a defined locus of the S. cerevisiae genome,
b) excising from the cloning vehicles obtained in a) fragments bearing the first recombination cassette (1) and the second recombination cassette (2), respectively, whereby each of the cassettes comprises the DNA sequence to be recombined (A, B) flanked by the respective two marker sequences, and each cassette (1, 2) in turn is flanked by targeting sequences,
c) transforming the fragments bearing the recombination cassettes (1,2) with flanking targeting sequences obtained in b) separately into S. cerevisiae diploid cells, whereby the targeting sequences direct the integration of the cassettes (1,2) into that locus to which they are homologous, in order to obtain diploid cells heterozygous for the first cassette (1), or the second cassette (2),
d) inducing separately the sporulation of the heterozygous diploid cells obtained in c) and
e) isolating haploid cells containing the first cassette (1) and expressing the first and second marker sequence and separately haploid cells containing the second cassette (2) and expressing the third and the fourth marker sequence.

10. Process according to claim 9, wherein the first cloning vehicle is plasmid pMXY9 having deposit number DSM 17010 and the second cloning vehicle is plasmid pMXY12 having deposit number DSM 17011.

11. Process according to any one of claims 4-6, 9 or 10, wherein the diploid S. cerevisiae cells used for transformation are auxotrophic for at least two nutritional factors.

12. Process according to claim 11, wherein the diploid cells are homozygous for the *ura3-1* allele and the *trp1-1* allele, which render them auxtrophic for uracil and tryptophan, respectively.

13. Process according to any one of claims 4-6 or 9-12, wherein the diploid cells used for transformation are resistant to at least two antibiotics.

14. Process according to claim 13, wherein the diploid cells are homozygous for the *can1-100* allele and the *cyh2R* allele, which render them resistant to canavanine and cycloheximide, respectively.

15. Process according to any one of claims 4-6 or 9-14, wherein diploid cells of the S. cerevisiae strain MXY47 having deposit number DSM 17026 are used for transformation, which are homozygous for the alleles *ura3-1, trp1-1, can1-100* and *cyh2R* and heterozygous for the *msh2::KanMX* mutation.

16. Process according to any one of claims 1 to 15, wherein the S. cerevisiae cells have a functional mismatch repair system.

17. Process according to any one of the claims 1 to 15, wherein the S. cerevisiae cells are transiently or permanently deficient in the mismatch repair system.

18. Process according to claim 17, wherein the transient or permanent deficiency of the mismatch repair system is due to a mutation and/or an inducible expression or repression of one or more genes involved in the mismatch repair system, a treatment with an agent that saturates the mismatch repair system and/or a treatment with an agent that globally impairs the mismatch repair.

19. Process according to any one of the claims 1 to 18, wherein the first (1) and the second (2) recombination cassettes are integrated in the *BUD31-HCM1* locus on chromosome III of the S. cerevisiae genome.

20. Process according to any one of claims 1 to 19, wherein the first and the second DNA sequences to be recombined (A, B) diverge by at least 1 nucleotide.

21. Process according to any one of claims 1 to 20, wherein the first and the second DNA sequences to be recombined (A, B) are derived from organisms other than S. cerevisiae.

22. Process according to any one of claims 1 to 21, wherein the first and the second DNA sequences to be recombined (A, B) comprise one or more non-coding sequences and/or one or more protein-coding sequences.

23. Process according to any of claims 1 to 22, wherein the marker sequences are selected from the group consisting of nutritional markers, pigment markers, antibiotic resistance markers, antibiotic sensitivity markers, primer recognition sites, intron/exon boundaries, sequences encoding a particular subunit of an enzyme, promoter sequences, downstream regulated gene sequences and restriction enzyme sites.

24. Process according to claim 23, where the first and the third marker sequences are nutritional markers, the gene products of which can compensate an auxotrophy of a S. cerevisiae cell.

25. Process according to claim 24, wherein the first marker sequence is *URA3,* the gene product of which can confer uracil prototrophy to an uracil auxotrophic S. cerevisiae cell.

26. Process according to claim 24, wherein the third marker sequence is *TRP1,* the gene product of which can confer tryptophan prototrophy to an tryptophan auxotrophic S. cerevisiae cell.

27. Process according to claim 23, wherein the second and fourth marker sequences are antibiotic sensitivity markers, the gene products of which can confer sensitivity to an antibiotic to a S. cerevisiae cell which is resistant to that antibiotic.

28. Process according to claim 27, wherein the second marker sequence is *CAN1,* the gene product of which can confer sensitivity to canavanine to a canavanine-resistant S. cerevisiae cell.

29. Process according to claim 27, wherein the fourth marker sequence is *CYH2,* the gene product of which can confer sensitivity to cycloheximide to a cycloheximide-resistant S. cerevisiae cell.

30. Process according to any one of claims 1 to 29, wherein haploid cells containing recombination cassettes with either first, second, third or fourth recombined DNA sequences are identified by PCR processes in order to detect the presence of the respective marker combination.

31. Process according to any one of claims 1 to 29, wherein haploid cells containing recombination cassettes with either firs, second, third or fourth recombined DNA sequences are identified by plating the haploid cells on media that select for the molecular linkage on the same DNA molecule of the respective marker combination.

32. Process according to claim 31, wherein haploid cells containing first recombined DNA sequences (A/B) are plated on a medium that selects for molecular linkage on the same DNA molecule of the first and the fourth marker sequences.

33. Process according to claim 31, wherein haploid cells containing second recombined DNA sequences (B/A) are plated on a medium that selects for molecular linkage on the same DNA molecule of the second and the third marker sequences.

34. Process according to claim 31, wherein haploid cells containing third recombined DNA sequences are plated on a medium that selects for molecular linkage on the same DNA molecule of the first and the second marker sequences.

35. Process according to claim 31, wherein haploid cells containing fourth recombined DNA sequences are plated on a medium that selects for molecular linkage on the same DNA molecule of the third and the fourth marker sequences.

36. Plasmid pMXY9, deposit number DSM 17010 comprising adjacently the *URA3* marker gene and the CAN1 marker gene, whereby the two marker sequences flank a polylinker sequence for inserting a DNA sequence to be recombined and whereby the two markers are flanked by targeting sequences homologous to the BUD31-HCM1 locus on chromosome III of the S. cerevisiae genome.

37. Plasmid pMXY9, deposit number DSM 17010, according to claim 36, wherein the polylinker sequence comprises restriction sites for the restriction enzymes *Sma*I, *Xba*I, *Pac*I and *Bgl*II.

38. Plasmid pMXY12, deposit number DSM 17011, comprising adjacently the *TRP1* marker gene and the *CYH2* marker gene, whereby the two marker sequences flank a polylinker sequence for inserting a DNA sequence to be re-combined (A, B) and whereby the two markers are flanked by targeting sequences homologous to the BUD31-HCM1 locus on chromosome III of the S. cerevisiae genome.

39. Plasmid pMXY12, deposit number DSM 17011, according to claim 38, wherein the polylinker sequence comprises restriction sites for the restriction enzymes *Sma*I, *Spe*I, and *Pac*I.

40. S. cerevisiae strain MXY47, deposit number DSM 17026, **characterized in that** diploid cells thereof are homozygous for the alleles *ura3-1, trp1-1, can1-100* and *cyh2R* and heterozygous for the *msh2::KanMX* mutation.

41. E.coli strain JM101, containing plasmid pMXY9, deposit number DSM 17010.

42. E.coli strain DH5a, containing plasmid pMXY12, deposit number DSM 17011.

43. Kit comprising at least a first container which comprises cells of S. cerevisiae strain MXY47, deposit number DSM 17026, a second container which comprises cells of E. coli strain JM101 containing plasmid pMXY9, deposit number DSM 17010, and a third container comprising cells of E. coli strain DH5a containing plasmid pMXY12, deposit number DSM 17011.

44. Kit comprising at least a first container comprising cells of S. cerevisiae strain MXY47, deposit number DSM 17026, a second container comprising DNA of plasmid pMXY9, deposit number DSM 17010, and a third container comprising DNA of plasmid pMXY12, deposit number DSM 17011.

## Patentansprüche

1. Verfahren zur Herstellung und zum Nachweis rekombinanter DNA-Sequenzen in Saccharomyces cerevisiae, das die Schritte umfasst:
a) Erzeugen von ersten diploiden S. cerevisiae-Zellen, die in einem bestimmten Lokus ihres Genoms eine erste Rekombinationskassette (1) tragen, die eine erste zu rekombinierende DNA-Sequenz (A) umfasst, die von mindestens einer ersten und einer zweiten Markersequenz flankiert ist, und in einer allelischen Position eine zweite Rekombinationskassette (2) tragen, die eine zweite zu rekombinierende DNA-Sequenz (B) umfasst, die von mindestens einer dritten und einer vierten Markersequenz flankiert ist,
b) Induzieren der Sporulation der in a) erhaltenen ersten diploiden Zellen
und
c) Isolieren haploider Zellen, die Rekombinationskassetten (3) enthalten, in denen erste rekombinierte DNA-Sequenzen (A/B) durch mindestens die erste und vierte Markersequenz flankiert sind, und haploider Zellen, die Rekombinationskassetten (4) enthalten, in denen zweite rekombinierte DNA-Sequenzen (B/A) durch mindestens die zweite und dritte Markersequenz flankiert sind.

2. Verfahren nach Anspruch 1, das weiter die Schritte umfasst:
a) Erzeugen zweiter diploider Zellen durch Paarung haploider Zellen, die die in 1c) erhaltenen ersten rekombinierten DNA-Sequenzen (A/B) enthalten, mit haploiden Zellen, die die in 1c) erhaltenen zweiten rekombinierten DNA-Sequenzen (B/A) enthalten,
b) Induzieren der Sporulation der in a) erhaltenen zweiten diploiden Zellen und
c) Isolieren haploider Zellen, die Rekombinationskassetten enthalten, in denen dritte rekombinierte DNA-Sequenzen durch mindestens die erste und zweite Markersequenz flankiert sind, und haploider Zellen, die vierte Rekombinationskassetten enthalten, in denen vierte rekombinierte DNA-Sequenzen durch mindestens die dritte und vierte Markersequenz flankiert sind.

3. Verfahren nach Anspruch 1 oder 2, wobei weitere rekombinierte DNA-Sequenzen durch Durchführen mindestens eines weiteren Paarungszyklus' der in 2c) erhaltenen haploiden Zellen mit anderen haploiden Zellen erzeugt werden, Induzieren der Sporulation der erhaltenen diploiden Zellen und Isolieren haploider Zellen mit rekombinierten DNA-Sequenzen auf der Basis der molekularen Verknüpfung zwischen zwei Markersequenzen.

4. Verfahren nach einem der Ansprüche 1 bis 3, wobei die erste diploide Zelle erzeugt wird durch simultane oder sequentielle Transformation einer diploiden S. cerevisiae-Zelle mit einem DNA-Molekül, das die erste Rekombinationskassette (1) enthält, und einem DNA-Molekül, das die zweite Rekombinationskassette (2) enthält, und Zulassen der Integration der zwei Rekombinationskassetten in allelische Positionen des S. cerevisiae-Genoms.

5. Verfahren nach Anspruch 4, wobei das DNA-Molekül, das die erste oder die zweite Rekombinationskassette (1,2) umfasst, ein künstliches Hefechromosom (YAC) ist.

6. Verfahren nach Anspruch 4, wobei das DNA-Molekül, das die erste oder die zweite Rekombinationskassette (1,2) umfasst, ein Clonierungsvehikel ist, wobei die entsprechenden zwei Markersequenzen durch Zielsequenzen flankiert sind, die homolog zu einem bestimmten Lokus des S. cerevisiae-Genoms sind.

7. Verfahren nach einem der Ansprüche 1 bis 3, wobei die erste diploide Zelle erzeugt wird durch Fusion einer haploiden S. cerevisiae-Zelle, die in einem Lokus ihres Genoms die erste Rekombinationskassette trägt, mit einer haploiden S. cerevisiae-Zelle, die in einer allelischen Position die zweite Rekombinationskassette trägt.

8. Verfahren nach einem der Ansprüche 1 bis 3, wobei die erste diploide Zelle erzeugt wird durch Paarung einer haploiden S. cerevisiae-Zelle, die in einem Lokus ihres Genoms die erste Rekombinationskassette trägt, mit einer haploiden S. cerevisiae-Zelle, die in einer allelischen Position die zweite Rekombinationskassette trägt.

9. Verfahren nach Anspruch 7 oder 8, wobei die haploiden Zellen, die die erste oder zweite Rekombinationskassette tragen, erzeugt werden durch:
a) Insertion der ersten zu rekombinierenden DNA-Sequenz zwischen der ersten und der zweiten Markersequenz, die benachbart auf einem ersten Clonierungsvehikel lokalisiert sind, und Insertion der zweiten zu rekombinierenden DNA-Sequenz zwischen der dritten und der vierten Markersequenz, die benachbart auf einem zweiten Clonierungsvehikel lokalisiert sind, wobei die entsprechenden zwei Markersequenzen von Zielsequenzen flankiert sind, die homolog zu einem bestimmten Lokus des S. cerevisiae-Genoms sind,
b) Ausschneiden von Fragmenten aus den in a) erhaltenen Clonierungsvehikeln, die die erste Rekombinationskassette (1) bzw. die zweite Rekombinationskassette (2) enthalten, wobei jede der Kassetten die zu rekombinierende DNA-Sequenz (A,B), flankiert durch die entsprechenden zwei Markersequenzen, umfasst und jede Kassette (1,2) wiederum durch Zielsequenzen flankiert ist,
c) separates Transformieren der in b) erhaltenen Fragmente, die die mit Zielsequenzen flankierten Rekombinationskassetten (1,2) enthalten, in diploide S. cerevisiae-Zellen, wobei die Zielsequenzen die Integration der Kassetten (1,2) in den Lokus, zu dem sie homolog sind, lenken, um diploide Zellen zu erhalten, die heterozygot für die erste Kassette (1) oder die zweite Kassette (2) sind,
d) separates Induzieren der Sporulation der in c) erhaltenen heterozygoten diploiden Zellen und
e) Isolieren haploider Zellen, die die erste Kassette (1) enthalten und die erste und zweite Markersequenz exprimieren, und separat haploider Zellen, die die zweite Kassette (2) enthalten und die dritte und vierte Markersequenz exprimieren.

10. Verfahren nach Anspruch 9, wobei das erste Clonierungsvehikel das Plasmid pMXY9 ist, das die Hinterlegungsnummer DSM 17010 hat, und das zweite Clonierungsvehikel das Plasmid pMXY12 ist, das die Hinterlegungsnummer DSM 17011 hat.

11. Verfahren nach einem der Ansprüche 4-6, 9 oder 10, wobei die diploiden S. cerevisiae-Zellen, die für die Transformation verwendet werden, auxotroph für mindestens zwei Ernährungsfaktoren sind.

12. Verfahren nach Anspruch 11, wobei die diploiden Zellen homozygot für das ura-3-1-Allel und das *trp1-1*-Allel sind, was sie auxotroph für Uracil bzw. Tryptophan macht.

13. Verfahren nach einem der Ansprüche 4-6 oder 9-12, wobei die diploiden Zellen, die für die Transformation verwendet werden, resistent gegenüber mindestens zwei Antibiotika sind.

14. Verfahren nach Anspruch 13, wobei die die diploiden Zellen homozygot für das *can1-100*-Allel und das *cyh2R*-Allel sind, was sie resistent gegenüber Canavanin bzw. Cycloheximid macht.

15. Verfahren nach einem der Ansprüche 4-6, oder 9-14, wobei die die diploiden Zellen des S. cerevisiae-Stamms MXY47, der die Hinterlegungsnummer DSM 17026 hat, zur Transformation verwendet werden, die homozygot für die Allele *ura-3-1, trp1-1, can1-100* und *cyh2R* und heterozygot für die *msh2::KanMX-*Mutation sind.

16. Verfahren nach einem der Ansprüche 1 bis 15, wobei die S. cerevisiae-Zellen ein funktionierendes Fehlpaarungsreparatursystem haben.

17. Verfahren nach einem der Ansprüche 1 bis 15, wobei die S. cerevisiae-Zellen vorübergehend oder permanent ein mangelhaftes Fehlpaarungsreparatursystem haben.

18. Verfahren nach Anspruch 17, wobei der vorübergehende oder permanente Mangel des Fehlpaarungsreparatursystem bedingt ist durch eine Mutation und/oder induzierbare Expression oder Repression von einem oder mehreren Genen, die in das Fehlpaarungsreparatursystem involviert sind, eine Behandlung mit einem Agens, das das Fehlpaarungsreparatursystem absättigt und/oder eine Behandlung mit einem Agens, das die Fehlpaarungsreparatur global beeinträchtigt.

19. Verfahren nach einem der Ansprüche 1 bis 18, wobei die erste (1) und die zweite (2) Rekombinationskassette in den *BUD31*-*HCM1*-Lokus auf Chromosom III des S. cerevisiae-Genoms integriert sind.

20. Verfahren nach einem der Ansprüche 1 bis 19, wobei die erste und die zweite zu rekombinierende DNA-Sequenz (A, B) durch mindestens 1 Nucleotid voneinander abweichen.

21. Verfahren nach einem der Ansprüche 1 bis 20, wobei die erste und die zweite zu rekombinierende DNA-Sequenz (A, B) von anderen Organismen als von S. cerevisiae abstammen.

22. Verfahren nach einem der Ansprüche 1 bis 21, wobei die erste und die zweite zu rekombinierende DNA-Sequenz (A, B) eine oder mehrere nicht-codierende Sequenzen und/oder eine oder mehrere Protein codierende Sequenzen umfassen.

23. Verfahren nach einem der Ansprüche 1 bis 22, wobei die Markersequenzen ausgewählt sind aus der Gruppe bestehend aus Ernährungsmarkern, Pigmentmarkern, Antibiotikaresistenzmarkern, Antibiotikasensitivitätsmarkern, Primererkennungsstellen, Intron-/Exongrenzen, Sequenzen, die eine bestimmte Untereinheit eines Enzyms codieren, Promotorsequenzen, stromabwärts regulierten Gensequenzen und Restriktionsenzymstellen.

24. Verfahren nach Anspruch 23, wobei die erste und die dritte Markersequenz Ernährungsmarker sind, deren Genprodukte eine Auxotrophie einer S. cerevisiae-Zelle kompensieren können.

25. Verfahren nach Anspruch 24, wobei die erste Markersequenz *URA3* ist, deren Genprodukt einer Uracil-auxotrophen S. cerevisiae-Zelle Uracil-Prototrophie verleihen kann.

26. Verfahren nach Anspruch 24, wobei die dritte Markersequenz *TRP1* ist, deren Genprodukt einer Tryptophan-auxotrophen S. cerevisiae-Zelle Tryptophan-Prototrophie verleihen kann.

27. Verfahren nach Anspruch 23, wobei die zweite und die vierte Markersequenz Antibiotikasensitivitätsmarker sind, deren Genprodukte einer S. cerevisiae-Zelle, die resistent gegen ein Antibiotikum ist, Sensitivität gegenüber diesem Antibiotikum verleihen kann.

28. Verfahren nach Anspruch 27, wobei die zweite Markersequenz *CAN1* ist, deren Genprodukt einer Canavanin-resistenten S. cerevisiae-Zelle Sensitivität gegenüber Canavanin verleihen kann.

29. Verfahren nach Anspruch 27, wobei die vierte Markersequenz *CYH2* ist, deren Genprodukt einer Cycloheximid-resistenten S. cerevisiae-Zelle Sensitivität gegenüber Cycloheximid verleihen kann.

30. Verfahren nach einem der Ansprüche 1 bis 29, wobei haploide Zellen, die Rekombinationskassetten mit entweder der ersten, zweiten, dritten oder vierten rekombinierten DNA-Sequenz enthalten, durch PCR-Verfahren identifiziert werden, um die Anwesenheit der jeweiligen Markerkombination nachzuweisen.

31. Verfahren nach einem der Ansprüche 1 bis 29, wobei haploide Zellen, die Rekombinationskassetten mit entweder der ersten, zweiten, dritten oder vierten rekombinierten DNA-Sequenz enthalten, durch Ausplattieren der haploiden Zellen auf Medien identifiziert werden, die auf die molekulare Verknüpfung der jeweiligen Markerkombination auf dem gleichen DNA-Molekül selektieren.

32. Verfahren nach Anspruch 31, wobei haploide Zellen, die erste rekombinierte DNA-Sequenzen enthalten (A/B), auf einem Medium ausplattiert werden, das auf die molekulare Verknüpfung der ersten und der vierten Markersequenz auf dem gleichen DNA-Molekül selektiert.

33. Verfahren nach Anspruch 31, wobei haploide Zellen, die zweite rekombinierte DNA-Sequenzen enthalten (B/A), auf einem Medium ausplattiert werden, das auf die molekulare Verknüpfung der zweiten und der dritten Markersequenz auf dem gleichen DNA-Molekül selektiert.

34. Verfahren nach Anspruch 31, wobei haploide Zellen, die dritte rekombinierte DNA-Sequenzen enthalten, auf einem Medium ausplattiert werden, das auf die molekulare Verknüpfung der ersten und der zweiten Markersequenz auf dem gleichen DNA-Molekül selektiert.

35. Verfahren nach Anspruch 31, wobei haploide Zellen, die vierte rekombinierte DNA-Sequenzen enthalten, auf einem Medium ausplattiert werden, das auf die molekulare Verknüpfung der dritten und der vierten Markersequenz auf dem gleichen DNA-Molekül selektiert.

36. Plasmid pMXY9, Hinterlegungsnummer DSM 17010, das benachbart das *URA3*-Markergen und das *CAN1*-Markergen umfasst, wobei die zwei Markersequenzen eine Polylinkersequenz zur Insertion einer zu rekombinierenden DNA-Sequenz flankieren und wobei die zwei Marker von Zielsequenzen flankiert sind, die homolog zu dem *BUD31*-*HCM1*-Lokus auf Chromosom III des S. cerevisiae-Genoms sind.

37. Plasmid pMXY9, Hinterlegungsnummer DSM 17010, nach Anspruch 36, wobei die Polylinkersequenz Restriktionsstellen für die Restriktionsenzyme *Sma*I, *Xba*I, *Pac*I und *Bgl*II umfasst.

38. Plasmid pMXY12, Hinterlegungsnummer DSM 17011, das benachbart das *TRP1*-Markergen und das *CYH2*-Markergen umfasst, wobei die zwei Markersequenzen eine Polylinkersequenz zur Insertion einer zu reombinierenden DNA-Sequenz (A, B) flankieren und wobei die zwei Marker von Zielsequenzen flankiert sind, die homolog zu dem *BUD31*-*HCM1*-Lokus auf Chromosom III des S. cerevisiae-Genoms sind.

39. Plasmid pMXY12, Hinterlegungsnummer DSM 17011, nach Anspruch 38, wobei die Polylinkersequenz Restriktionsstellen für die Restriktionsenzyme *Sma*I, *Spe*I und *Pac*I umfasst.

40. S. cerevisiae-Stamm MXY47, Hinterlegungsnummer DSM 17026, **dadurch gekennzeichnet, dass** diploide Zellen davon homozygot für die Allele *ura3-1*, *trp1-1, can1-100* und *cyh2R* und heterozygot für die *msh2::KanMX*-Mutation sind.

41. E. coli-Stamm JM101, der Plasmid pMXY9, Hinterlegungsnummer DSM 17010, enthält.

42. E. coli-Stamm DH5a, der Plasmid pMXY12, Hinterlegungsnummer DSM 17011, enthält.

43. Kit, das mindestens einen ersten Behälter umfasst, der Zellen des S. cerevisiae-Stamms MXY47, Hinterlegungsnummer DSM 17026, umfasst, einen zweiten Behälter, der Zellen des E. coli-Stamms JM101 umfasst, der Plasmid pMXY9, Hinterlegungsnummer DSM 17010, enthält, und einen dritten Behälter, der Zellen des E. coli-Stamms DH5a umfasst, der Plasmid pMXY12, Hinterlegungsnummer DSM 17011, enthält.

44. Kit, das mindestens einen ersten Behälter umfasst, der Zellen des S. cerevisiae-Stamms MXY47, Hinterlegungsnummer DSM 17026, umfasst, einen zweiten Behälter, der DNA des Plasmids pMXY9, Hinterlegungsnummer DSM 17010, umfasst, und einen dritten Behälter, der DNA des Plasmids pMXY12, Hinterlegungsnummer DSM 17011, umfasst.

## Revendications

1. Procédé pour engendrer et détecter des séquences d'ADN recombinant dans Saccharomyces *cerevisiae,* comprenant les étapes consistant :
a) à engendrer des premières cellules de *S. cerevisiae* diploïdes portant dans un locus défini de leur génome une première cassette de recombinaison (1) comprenant une première séquence d'ADN à recombiner (A), qui est flanquée par au moins des première et deuxième séquence de marqueur, et, en une position allélique, une seconde cassette de recombinaison (2) comprenant une seconde séquence d'ADN à recombiner (B), qui est flanquée par au moins des troisième et quatrième séquence de marqueur,
b) à induire la sporulation des premières cellules diploïdes obtenues en a), et
c) à isoler les cellules haploïdes contenant les cassettes de recombinaison (3) dans lesquelles les premières séquences d'ADN recombinées (A/B) sont flanquées par au moins les première et quatrième séquences de marqueur, et les cellules haploïdes contenant des cassettes de recombinaison (4) dans lesquelles les secondes séquences d'ADN recombinées (B/A) sont flanquées par au moins les deuxième et troisième séquences de marqueur.

2. Procédé suivant la revendication 1, comprenant en outre les étapes consistant :
a) à engendrer des secondes cellules diploïdes par accouplement des cellules haploïdes contenant les premières séquences d'ADN recombinées (A/B) obtenues en 1c) avec les cellules haploïdes contenant les secondes séquences d'ADN recombinées (B/A) obtenues en 1c),
b) à induire la sporulation des secondes cellules diploïdes obtenues en a), et
c) à isoler les cellules haploïdes contenant les cassettes de recombinaison dans lesquelles les troisièmes séquences d'ADN recombinées sont flanquées par au moins les première et deuxième séquences de marqueur, et les cellules haploïdes contenant les quatrièmes cassettes de recombinaison dans lesquelles les quatrièmes séquences d'ADN recombinées sont flanquées par au moins les troisième et quatrième séquences de marqueur.

3. Procédé suivant la revendication 1 ou 2, dans lequel des séquences d'ADN recombinées supplémentaires sont engendrées en soumettant les cellules haploïdes obtenues en 2c) au moins une fois à un autre cycle d'accouplement avec d'autres cellules haploïdes, en induisant la sporulation des cellules diploïdes obtenues et en isolant les cellules haploïdes avec les séquences d'ADN recombinées sur la base de la liaison moléculaire entre les deux séquences de marqueur.

4. Procédé suivant l'une quelconque des revendications 1 à 3, dans lequel la première cellule diploïde est engendrée en transformant simultanément ou séquentiellement une cellule de *S. cerevisiae* diploïde avec une molécule d'ADN contenant la première cassette de recombinaison (1) et une molécule d'ADN contenant la seconde cassette de recombinaison (2) et en laissant s'effectuer l'intégration des deux cassettes de recombinaison à des positions alléliques du génome de *S. cerevisiae.*

5. Procédé suivant la revendication 4, dans lequel la molécule d'ADN comprenant la première ou seconde cassette de recombinaison (1, 2) est un chromosome artificiel de levure (YAC).

6. Procédé suivant la revendication 4, dans lequel la molécule d'ADN comprenant la première ou seconde cassette de recombinaison est un vecteur de clonage, les deux séquences de marqueur respectives étant ainsi flanquées par des séquences de ciblage qui sont homologues d'un locus défini du génome de *S. cerevisiae.*

7. Procédé suivant l'une quelconque des revendications 1 à 3, dans lequel la première cellule diploïde est engendrée par fusion d'une cellule de *S. cerevisiae* haploïde portant dans un locus de son génome la première cassette de recombinaison avec une cellule de *S. cerevisiae* haploïde portant à une position allélique la seconde cassette de recombinaison.

8. Procédé suivant l'une quelconque des revendications 1 à 3, dans lequel la première cellule diploïde est engendrée par accouplement d'une cellule de *S. cerevisiae* haploïde portant dans un locus de son génome la première cassette de recombinaison avec une cellule de *S. cerevisiae* haploïde portant à une position allélique la seconde cassette de recombinaison.

9. Procédé suivant la revendication 7 ou 8, dans lequel des cellules diploïdes portant la première ou seconde cassette de recombinaison sont engendrées :
a) en insérant la première séquence d'ADN à recombiner entre les première et deuxième séquences de marqueur situées en positions adjacentes sur un premier vecteur de clonage et en insérant la seconde séquence d'ADN à recombiner entre les troisième et quatrième séquences de marqueur situées en positions adjacentes sur un second vecteur de clonage, les deux séquences de marqueur respectives étant ainsi flanquées par des séquences de ciblage qui sont homologues d'un locus défini du génome de *S. cerevisiae.*
b) en excisant des vecteurs de clonage obtenus en a) les fragments portant la première cassette de recombinaison (1) et la seconde cassette de recombinaison (2), respectivement, chacune des cassettes comprenant ainsi la séquence d'ADN à recombiner (A, B) flanquée par les deux séquences de marqueur respectives, et chaque cassette (1, 2) étant alors flanquée par les séquences de ciblage,
c) en transformant les fragments portant les cassettes de recombinaison (1,2) avec des séquences de ciblage adjacentes obtenues en b) séparément dans des cellules diploïdes de *S. cerevisiae,* les séquences de ciblage dirigeant ainsi l'intégration des cassettes (1,2) dans le locus avec lequel elles présentent une homologie, afin d'obtenir des cellules diploïdes hétérozygotes pour la première cassette (1) ou la seconde cassette (2),
d) en induisant séparément la sporulation des cellules diploïdes hétérozygotes obtenues en c), et
e) en isolant les cellules haploïdes contenant la première cassette (1) et en exprimant les première et deuxième séquences de marqueur et, séparément, les cellules haploïdes contenant la seconde cassette (2) et en exprimant les troisième et quatrième séquences de marqueur.

10. Procédé suivant la revendication 9, dans lequel le premier vecteur de clonage est le plasmide pMXY9 ayant le numéro de dépôt DSM 17010 et le second vecteur de clonage est le plasmide pMXY12 ayant le numéro de dépôt DSM 17011.

11. Procédé suivant l'une quelconque des revendications 4-6, 9 ou 10, dans lequel les cellules de *S. cerevisiae* diploïdes utilisées pour la transformation sont auxotrophes pour au moins deux facteurs nutritionnels.

12. Procédé suivant la revendication 11, dans lequel les cellules diploïdes sont homozygotes pour l'allèle *ura3-1* et l'allèle *trp1-1,* qui les rendent auxotrophes respectivement pour l'uracile et le tryptophane.

13. Procédé suivant l'une quelconque des revendications 4-6 ou 9-12, dans lequel les cellules diploïdes utilisées pour la transformation sont résistantes à au moins deux antibiotiques.

14. Procédé suivant la revendication 13, dans lequel les cellules diploïdes sont homozygotes pour l'allèle *can1-100* et l'allèle *cyh2R,* qui les rendent résistantes respectivement à la canavanine et au cycloheximide.

15. Procédé suivant l'une quelconque des revendications 4-6 ou 9-14, dans lequel sont utilisées pour la transformation, des cellules diploïdes de la souche *S. cerevisiae* MXY47 ayant le numéro de dépôt DSM 17026, qui sont homozygotes pour les allèles *ura3-1, trp1-1, can1-100* et *cyh2R* et hétérozygotes pour la mutation *msh2::KanMX.*

16. Procédé suivant l'une quelconque des revendications 1 à 15, dans lequel les cellules de *S. cerevisiae* ont un système fonctionnel de réparation des mésappariements.

17. Procédé suivant l'une quelconque des revendications 1 à 15, dans lequel les cellules de *S. cerevisiae* sont déficientes de manière transitoire ou permanente en le système de réparation des mésappariements.

18. Procédé suivant la revendication 17, dans lequel la déficience transitoire ou permanente en le système de réparation des mésappariements est due à une mutation et/ou expression ou répression inductible d'un ou plusieurs gènes impliqués dans le système de réparation des mésappariements, un traitement avec un agent qui sature le système de réparation des mésappariements et/ou un traitement avec un agent qui entrave globalement la réparation des mésappariements.

19. Procédé suivant l'une quelconque des revendications 1 à 18, dans lequel les première (1) et seconde (2) cassettes de recombinaison sont intégrées au locus *BUD31-HCM1* sur le chromosome III du génome de *S. cerevisiae.*

20. Procédé suivant l'une quelconque des revendications 1 à 19, dans lequel les première et seconde séquences d'ADN à recombiner (A, B) divergent d'au moins 1 nucléotide.

21. Procédé suivant l'une quelconque des revendications 1 à 20, dans lequel les première et seconde séquences d'ADN à recombiner (A, B) sont dérivées d'organismes autres que *S. cerevisiae.*

22. Procédé suivant l'une quelconque des revendications 1 à 21, dans lequel les première et seconde séquences d'ADN à recombiner (A, B) comprennent une ou plusieurs séquences non codantes et/ou une ou plusieurs séquences codant pour les protéines.

23. Procédé suivant l'une quelconque des revendications 1 à 22, dans lequel des séquences de marqueur sont choisies dans le groupe consistant en des marqueurs nutritionnels, des marqueurs consistant en pigments, des marqueurs de résistance à des antibiotiques, des marqueurs de sensibilité à des antibiotiques, des sites de reconnaissance d'amorces, des limites intron/exon, des séquences codant pour une sous-unité particulière d'une enzyme, des séquences de promoteurs, des séquences de gènes régulées en aval et des sites d'enzymes de restriction.

24. Procédé suivant la revendication 23, dans lequel les première et troisième séquences de marqueur sont des marqueurs nutritionnels, dont les produits de gènes peuvent compenser une auxotrophie d'une cellule de *S. cerevisiae.*

25. Procédé suivant la revendication 24, dans lequel la première séquence de marqueur est la séquence *URA3,* dont le produit de gène peut conférer une prototrophie pour l'uracile à une cellule de *S. cerevisiae* auxotrophe pour l'uracile.

26. Procédé suivant la revendication 24, dans lequel la troisième séquence de marqueur est la séquence *TRP1*, dont le produit de gène peut conférer une prototrophie pour le tryptophane à une cellule de S. *cerevisiae* auxotrophe pour le tryptophane.

27. Procédé suivant la revendication 23, dans lequel les deuxième et quatrième séquences de marqueur sont des marqueurs de sensibilité à des antibiotiques, dont les produits de gènes peuvent conférer une sensibilité à un antibiotique à une cellule de *S. cerevisiae* qui est résistante à cet antibiotique.

28. Procédé suivant la revendication 27, dans lequel la deuxième séquence de marqueur est la séquence *CAN1*, dont le produit de gène peut conférer une sensibilité à la canavanine à une cellule de *S. cerevisiae* résistante à la canavanine.

29. Procédé suivant la revendication 27, dans lequel la quatrième séquence de marqueur est la séquence *CYH2,* dont le produit de gène peut conférer une sensibilité au cycloheximide à une cellule de *S. cerevisiae* résistante au cycloheximide.

30. Procédé suivant l'une quelconque des revendications 1 à 29, dans lequel des cellules haploïdes contenant des cassettes de recombinaison avec des premières, deuxièmes, troisièmes ou quatrièmes séquences d'ADN recombinées sont identifiées par des procédés de PCR afin de détecter la présence de la combinaison respective de marqueurs.

31. Procédé suivant l'une quelconque des revendications 1 à 29, dans lequel des cellules haploïdes contenant des cassettes de recombinaison avec des premières, deuxièmes, troisièmes ou quatrièmes séquences d'ADN recombinées sont identifiées par étalement des cellules haploïdes sur des milieux qui sélectionnent la liaison moléculaire sur la même molécule d'ADN de la combinaison respective de marqueurs.

32. Procédé suivant la revendication 31, dans lequel des cellules haploïdes contenant des premières séquences d'ADN recombinées (A/B) sont étalées sur un milieu qui sélectionne la liaison moléculaire sur la même molécule d'ADN des première et quatrième séquences de marqueur.

33. Procédé suivant la revendication 31, dans lequel des cellules haploïdes contenant des deuxièmes séquences d'ADN recombinées (B/A) sont étalées sur un milieu qui sélectionne la liaison moléculaire sur la même molécule d'ADN des deuxième et troisième séquences de marqueur.

34. Procédé suivant la revendication 31, dans lequel des cellules haploïdes contenant des troisièmes séquences d'ADN recombinées sont étalées sur un milieu qui sélectionne la liaison moléculaire sur la même molécule d'ADN des première et deuxième séquences de marqueur.

35. Procédé suivant la revendication 31, dans lequel des cellules haploïdes contenant des quatrièmes séquences d'ADN recombinées sont étalées sur un milieu qui sélectionne la liaison moléculaire sur la même molécule d'ADN des troisième et quatrième séquences de marqueur.

36. Plasmide pMXY9, numéro de dépôt DSM 17010, comprenant de manière adjacente le gène de marqueur *URA3* et le gène de marqueur *CAN1*, les deux séquences de marqueur flanquant ainsi une séquence de polylinker pour l'insertion d'une séquence d'ADN à recombiner, et les deux marqueurs étant ainsi flanqués par des séquences de ciblage homologues du locus BUD31-HCM1 sur le chromosome III du génome de *S. cerevisiae.*

37. Plasmide pMXY9, numéro de dépôt DSM 17010, suivant la revendication 36, dans lequel la séquence de polylinker comprend des sites de restriction pour les enzymes de restriction *Sma*I, *Xba*I, *Pac*I et *Bg*/II.

38. Plasmide pMXY12, numéro de dépôt DSM 17011, comprenant de manière adjacente le gène de marqueur *TRP1* et le gène de marqueur *CYH2,* les deux séquences de marqueur flanquant ainsi une séquence de polylinker pour l'insertion d'une séquence d'ADN à recombiner (A, B), et les deux marqueurs étant ainsi flanqués par des séquences de ciblage homologues du locus BUD31-HCM1 sur le chromosome III du génome de *S. cerevisiae.*

39. Plasmide pMXY12, numéro de dépôt DSM 17011, suivant la revendication 38, dans lequel la séquence de polylinker comprend des sites de restriction pour les enzymes de restriction *Sma*l, *Spe*l et *Pac*l.

40. Souche *S. cerevisiae* MXY47, numéro de dépôt DSM 17026, **caractérisée en ce que** ses cellules diploïdes sont homozygotes pour les allèles *ura3-1, trp1-1, can1-100* et *cyh2R* et hétérozygotes pour la mutation *msh2::KanMX.*

41. Souche E.coli JM101, contenant le plasmide pMXY9, numéro de dépôt DSM 17010.

42. Souche E.coli DH5a, contenant le plasmide pMXY12, numéro de dépôt DSM 17011.

43. Kit comprenant au moins un premier récipient qui comprend des cellules de la souche *S. cerevisiae* MXY47, numéro de dépôt DSM 17026, un deuxième récipient qui comprend des cellules de la souche *E. coli* JM101 contenant le plasmide pMXY9, numéro de dépôt DSM 17010 et un troisième récipient comprenant des cellules de la souche *E. coli* DH5a contenant le plasmide pMXY12, numéro de dépôt DSM 17011.

44. Kit comprenant au moins un premier récipient comprenant des cellules de la souche *S. cerevisiae* MXY47, numéro de dépôt DSM 17026, un deuxième récipient comprenant de l'ADN du plasmide pMXY9, numéro de dépôt DSM 17010 et un troisième récipient comprenant de l'ADN du plasmide pMXY12, numéro de dépôt DSM 17011.


### SEQUENCE LISTING

<110> Mixis France S.A.
<120> Generation of recombinant genes in Saccharomyces cerevisiae
<130> 18248
<140>
   <141>
<160> 33
<170> PatentIn Ver. 2.1
<210> 1
   <211> 20
   <212> DNA
   <213> Saccharomyces cerevisiae
<400> 1 agaactccag cactccgtat 20
<210> 2
   <211> 20
   <212> DNA
   <213> Saccharomyces cerevisiae
<400> 2 gagacctatc gccatcttca 20
<210> 3
   <211> 20
   <212> DNA
   <213> Saccharomyces cerevisiae
<400> 3 tcggttctta ctgccaagtg 20
<210> 4
   <211> 20
   <212> DNA
   <213> Saccharomyces cerevisiae
<400> 4 gagtggcaag aacttgtagc 20
<210> 5
   <211> 20
   <212> DNA
   <213> Saccharomyces cerevisiae
<400> 5 catgacgatg aggacatcac 20
<210> 6
   <211> 20
   <212> DNA
   <213> Saccharomyces cerevisiae
<400> 6 cgttcggacc taacatctct 20
<210> 7
   <211> 20
   <212> DNA
   <213> Saccharomyces cerevisiae
<400> 7 atgcttgatg gtcggaagag 20
<210> 8
   <211> 20
   <212> DNA
   <213> Saccharomyces cerevisiae
<400> 8 tatgcctctt ccgaccatca 20
<210> 9
   <211> 20
   <212> DNA
   <213> Saccharomyces cerevisiae
<400> 9 aggtcgcctg acgcatatac 20
<210> 10
   <211> 20
   <212> DNA
   <213> Saccharomyces cerevisiae
<400> 10 tgaagaggag gtcgactacg 20
<210> 11
   <211> 20
   <212> DNA
   <213>. Saccharomyces cerevisiae
<400> 11 ccacctagcg gatgactctt 20
<210> 12
   <211> 20
   <212> DNA
   <213> Saccharomyces cerevisiae
<400> 12 cgcctcgttc agaatgacac 20
<210> 13
   <211> 45
   <212> DNA
   <213> Saccharomyces cerevisiae
<400> 13 acctctagat cttaattaag ttaacttacg gtgccctttg atcct 45
<210> 14
   <211> 31
   <212> DNA
   <213> Saccharomyces cerevisiae
<400> 14 caagcggccg cagcgcatgt gtccgatctt t 31
<210> 15
   <211> 39
   <212> DNA
   <213> Saccharomyces cerevisiae
<400> 15 caaggtacca gcggccgctg tgctgtctgc gctgcattc 39
<210> 16
   <211> 28
   <212> DNA
   <213> Saccharomyces cerevisiae
<400> 16 acactcgagg caccgtaagg gaggagaa 28
<210> 17
   <211> 39
   <212> DNA
   <213> Saccharomyces cerevisiae
<400> 17 caaggtacca gcggccgctg tgctgtctgc gctgcattc 39
<210> 18
   <211> 36
   <212> DNA
   <213> Saccharomyces cerevisiae
<400> 18 acctctagac tagtcgaccc aaggaggtgc catgaa 36
<210> 19
   <211> 35
   <212> DNA
   <213> Saccharomyces cerevisiae
<400> 19 accttaatta agtcgacctg cagcgaattg ttagc 35
<210> 20
   <211> 31
   <212> DNA
   <213> Saccharomyces cerevisiae
<400> 20 accactagtc gacaccaaga aggtgccatg a 31
<210> 21
   <211> 35
   <212> DNA
   <213> Saccharomyces cerevisiae
<400> 21 accttaatta agtcgacggt cacgatgctg tactt 35
<210> 22
   <211> 20
   <212> DNA
   <213> Saccharomyces cerevisiae
<400> 22 tcattgcctt ctccactctc 20
<210> 23
   <211> 20
   <212> DNA
   <213> Saccharomyces cerevisiae
<400> 23 caccagcgct ctagatacat 20
<210> 24
   <211> 20
   <212> DNA
   <213> Saccharomyces cerevisiae
<400> 24 ggaccagaac tacctgtgaa 20
<210> 25
   <211> 20
   <212> DNA
   <213> Saccharomyces cerevisiae
<400> 25 gagtagcagc acgttcctta 20
<210> 26
   <211> 41
   <212> DNA
   <213> Saccharomyces cerevisiae
<400> 26 ccaggatccc gggttaatta agttcccagg aaaccctttc g 41
<210> 27
   <211> 28
   <212> DNA
   <213> Saccharomyces cerevisiae
<400> 27 caacgatcgg atctgccact ggtaactt 28
<210> 28
   <211> 20
   <212> DNA
   <213> Saccharomyces cerevisiae
<400> 28 ttgtttgtgc acttgcctgc 20
<210> 29
   <211> 20
   <212> DNA
   <213> Saccharomyces cerevisiae
<400> 29 aaagccgtat gactcaccca 20
<210> 30
   <211> 20
   <212> DNA
   <213> Saccharomyces cerevisiae
<400> 30 gaaagggttt cctgggaact 20
<210> 31
   <211> 37
   <212> DNA
   <213> Saccharomyces cerevisiae
<400> 31 cgcggatcca ctagtcgacc caccaaggta ccatgaa 37
<210> 32
   <211> 35
   <212> DNA
   <213> Saccharomyces cerevisiae
<400> 32 accttaatta agtcgaccga gccttgagcg ccttg 35
<210> 33
   <211> 20
   <212> DNA
   <213> Saccharomyces cerevisiae
<400> 33 tgagaagatg cggccagcaa 20